# EUROPEAN PATENT SPECIFICATION

(11) **EP 2 568 978 B1**
(45) Date of publication and mention of the grant of the patent: **24.04.2019**
(21) Application number: 11781359.2
(22) Date of filing: 13.05.2011
(51) Int. Cl.: A61K 31/635, A61K 31/195, A61K 31/198, A61K 31/355, A61K 31/13, A61P 35/00, A61K 31/192, A61K 45/06, A61K 31/415

(54) **CANCER PREVENTION AND TREATMENT BASED ON DIETARY POLYAMINE CONTENT**
KREBSVORBEUGUNG UND -BEHANDLUNG BASIEREND AUF POLYAMIN-NAHRUNGSMITTELGEHALT
PRÉVENTION ET TRAITEMENT DU CANCER FONDÉES SUR LA TENEUR EN POLYAMINES PROVENANT DE L'ALIMENTATION

(30) Priority: 14.05.2010 US 345048 P
(43) Date of publication of application: 20.03.2013
(73) Proprietor: Arizona Board of Regents on Behalf of University of Arizona, Tucson, AZ 85721 (US); The Regents of the University of California, A California Corporation, Oakland, CA 94607 (US); Cancer Prevention Pharmaceuticals, Inc., Tucson, AZ 85718 (US)
(72) Inventor: RAJ, Kavitha, P., Orange, CA 92868 (US); ZELL, Jason, Orange, CA 92868 (US); McLAREN, Christine, E., Irvine, CA 92697-7550 (US); GERNER, Eugene, W., Tucson, AZ 85704-1718 (US); MEYSKENS, Frank, L., Irvine, CA 92603 (US); JACOB, Jeffrey, Tucson, AZ 85718 (US)
(74) Representative: Potter Clarkson
(86) International application number: PCT/US2011/036464
(87) International publication number: WO 2011/143579

(56) References cited:
- WO-A1-99/49859
- WO-A2-02/15895
- CA-A1- 2 165 481
- U. K. BASUROY: "Emerging Concepts in Targeting the Polyamine Metabolic Pathway in Epithelial Cancer Chemoprevention and Chemotherapy", JOURNAL OF BIOCHEMISTRY, vol. 139, no. 1, 1 January 2006 (2006-01-01), pages 27-33, XP055032902, ISSN: 0021-924X, DOI: 10.1093/jb/mvj022
- QUEMENER V ET AL: "POLYAMINE DEPRIVATION: A NEW TOOL IN CANCER TREATMENT", ANTICANCER RESEARCH, INTERNATIONAL INSTITUTE OF ANTICANCER RESEARCH, GR, vol. 14, no. 2A, 1 March 1994 (1994-03-01) , pages 443-448, XP008014124, ISSN: 0250-7005
- NIKOLAUS SEILER ET AL: "Endogenous and Exogenous Polyamines in Support of Tumor Growth", CANCER RESEARCH, AMERICAN ASSOCIATION FOR CANCER RESEARCH, US, vol. 50, 15 August 1990 (1990-08-15), pages 5077-5083, XP007910864, ISSN: 0008-5472
- F. L. MEYSKENS ET AL: "Difluoromethylornithine Plus Sulindac for the Prevention of Sporadic Colorectal Adenomas: A Randomized Placebo-Controlled, Double-Blind Trial", CANCER PREVENTION RESEARCH, vol. 1, no. 1, 14 April 2008 (2008-04-14), pages 32-38, XP055085335, ISSN: 1940-6207, DOI: 10.1158/1940-6207.CAPR-08-0042
- E.W. GERNER: "Impact of dietary amino acids and polyamines on intestinal carcinogenesis and chemoprevention in mouse models", BIOCHEMICAL SOCIETY TRANSACTIONS, vol. 35, no. 2, 1 April 2007 (2007-04-01), page 322, XP055085416, ISSN: 0300-5127, DOI: 10.1042/BST0350322
- K P RAJ ET AL: "Role of dietary polyamines in a phase III clinical trial of difluoromethylornithine (DFMO) and sulindac for prevention of sporadic colorectal adenomas", BRITISH JOURNAL OF CANCER, vol. 108, no. 3, 22 January 2013 (2013-01-22), pages 512-518, XP055085332, ISSN: 0007-0920, DOI: 10.1038/bjc.2013.15

## Description

### BACKGROUND OF THE INVENTION

### I. Field of the Invention

The present invention relates generally to the fields of cancer biology and medicine. More particularly, it concerns the prevention and treatment of carcinomas and risk factors thereof.

### II. Description of Related Art

There remains a need for more effective methods for treating and preventing colorectal cancers and other carcinomas. According to the National Cancer Institute, there were approximately 147,000 new cases and 50,000 deaths from colorectal cancer in the United States in 2009. Current treatment protocols, especially those for colon cancers and polyps, include tumor resection, chemotherapy and radiation therapy.

Understanding key mechanisms that explain colorectal tumorigenesis should facilitate the development of new approaches to colorectal cancer prevention. Compelling experimental and epidemiologic studies indicate that diet and nutritional factors are important factors in modulating transformation of normal epithelium to frank carcinoma. Identifying dietary constituents with antitumor activity, and investigating their mechanism of action may lead to significant advances in cancer prevention, particularly within the colon. Linsalata M, Russo F: Nutritional factors and polyamine metabolism in colorectal cancer. Nutrition 24:382-9, 2008

Polyamines are organic cations found in every living cell. Wallace HM: Polyamines in human health. Proc Nutr Soc 55:419-31, 1996. They are synthesized endogenously from the arginine-derived product ornithine, by the rate-limiting enzyme ornithine decarboxylase (ODC). Gerner EW, Meyskens FL, Jr.: Polyamines and cancer: old molecules, new understanding. Nat Rev Cancer 4:781-92, 2004 Ornithine is converted by ODC to putrescine, which is sequentially converted into spermine and spermidine by SSAT (spermine spermidine acetyltranferase); these three molecules are interconvertible. A link between polyamines and cancer has been established for several decades. In addition to the well-described series of biochemical, genetic and epigenetic alterations involved in colorectal carcinogenesis, elevated epithelial polyamine content has been consistently implicated in CRC carcinogenesis. Gerner EW, Meyskens FL, Jr.: Polyamines and cancer: old molecules, new understanding. Nat Rev Cancer 4:781-92, 2004; Fearon ER, Vogelstein B: A genetic model for colorectal tumorigenesis. Cell 61:759-67, 1990; Jass JR, Whitehall VL, Young J, et al: Emerging concepts in colorectal neoplasia. Gastroenterology 123:862-76, 2002. Overexpression of ODC in the rectal mucosa has been associated with colorectal cancer risk and found to be a potential biochemical marker of proliferation in CRC. Wang W, Liu LQ, Higuchi CM: Mucosal polyamine measurements and colorectal cancer risk. J Cell Biochem 63:252-7, 1996; McGarrity TJ, Peiffer LP, Bartholomew MJ, et al.: Colonic polyamine content and ornithine decarboxylase activity as markers for adenomas. Cancer 66:1539-43, 1990; Brabender J, Lord RV, Danenberg KD, et al.: Upregulation of ornithine decarboxylase mRNA expression in Barrett's esophagus and Barrett's-associated adenocarcinoma. J Gastrointest Surg 5:174-81; discussion 182, 2001 Polyamines have been reported to regulate oncogene expression and function through transcriptional and posttranscriptional processes. Tabib A, Bachrach U: Role of polyamines in mediating malignant transformation and oncogene expression. Int J Biochem Cell Biol 31:1289-95, 1999; Bachrach U, Wang YC, Tabib A: Polyamines: new cues in cellular signal transduction. News Physiol Sci 16:106-9, 2001. Difluoromethylornithine (DFMO, eflornithine) irreversibly inhibits ODC which results in decreased polyamine synthesis. DFMO is a widely studied example of a polyamine-metabolism inhibitor that suppresses cancer development in animal models. Meyskens FL, Jr., Gerner EW: Development of difluoromethylornithine (DFMO) as a chemoprevention agent. Clin Cancer Res 5:945-51, 1999 and WO 99/49859, which was published on 26 March 1999 and is entitled "DFMO and Sulindac combination in cancer chemoprevention". Together with the NSAID sulindac, DFMO has been recently shown to decrease the incidence of metachronous colorectal adenomas in human clinical trials. Meyskens FL, McLaren C.E. , Pelot D., Fujikawa S., et al.: Difluoromethylornithine plus sulindac for the prevention of sporadic colorectal adenomas: a randomized placebo-controlled, double-blind trial. Cancer Prevention Research 1:32-38,2008. In addition to endogenous polyamine production, dietary polyamines and their metabolism by intestinal micro-organisms have been shown to be major determinants of the total body polyamine pool. Zoumas-Morse C, Rock CL, Quintana EL, et al.: Development of a polyamine database for assessing dietary intake. J Am Diet Assoc 107:1024-7, 2007. Notable dietary polyamines include spermine, spermidine and putrescine. Polyamine absorption occurs in the gut, and then the various forms of polyamines are metabolized in tissues under the strict regulation of ODC. Thomas T, Thomas TJ: Polyamine metabolism and cancer. J Cell Mol Med 7:113-26, 2003. Despite extensive evidence about dietary polyamines, it is unknown whether dietary polyamines influence tissue polyamines or adenoma formation in humans. Determining whether and how dietary polyamine intake may affects cancer risk factors, and whether or how it should factor into to preventative and curative treatment protocols would be a major advantage.

### SUMMARY OF THE INVENTION

According to the present invention, there is provided a composition according to Claims 1 to 3 for use in the prevention or treatment of carcinoma in a patient, wherein the patient is as defined in Claims 1 to 3. According to the present invention, there is also provided a method of selecting a patient for suitability for treatment of carcinoma according to Claim 13.

Also described herein, is a method for predicting the efficacy of an anti-carcinoma combination therapy comprising an ornithine decarboxylase (ODC) inhibitor and an agent that modulates the polyamine pathway to reduce overall cellular polyamine content comprising assessing a tissue polyamine level or tissue polyamine flux in a patient to be treated with said combination therapy, wherein a high tissue polyamine level or flux predicts a lower efficacy for said treatment.

The agent that modulates the polyamine pathway to reduce overall cellular polyamine content can be a spermidine/spermine *N*¹-acetyltransferase expression agonist. Said tissue polyamine level or tissue polyamine flux can be determined from rectal mucosal tissue, prostate tissue or urine.

The method described herein further comprises treating said patient with said combination therapy if said tissue polyamine level or tissue polyamine flux is not high. The method can further comprise subjecting said patient to a low polyamine diet prior to or at the time of commencing said combination therapy. The method can also comprise obtaining results of a test that determines said patient's genotype at position +316 of at least one *ODC1* promoter gene allele.

As described herein, said results can be obtained by receiving a report containing said genotype or taking a patient history that reveals said genotype. In some embodiments, said test can be used to determine the nucleotide base at position +316 of one allele of the *ODC1* promoter gene of the patientor said test determines the nucleotide bases at position +316 of both alleles of the *ODC1* promoter gene of the patient.

The ornithine decarboxylase (ODC) inhibitor for use in accordance with the present invention is α-difluoromethylornithine (DFMO), and is used in a combined effective amount with a spermidine/spermine N¹-acetyltransferase expression agonist that is a non-aspirin containing non-steroidal anti-inflammatory drug (NSAID) as described in Claims 1 to 3.

In one embodiment, the non-aspirin containing NSAID is a selective COX-2 inhibitor. In embodiment, the non-aspirin containing NSAID is sulindac or celecoxib. In embodiment, the non-aspirin containing NSAID is sulindac. In embodiment, the non-aspirin containing NSAID is celecoxib.

Also described herein is a method for predicting the efficacy of an anti-carcinoma combination therapy comprising an ornithine decarboxylase (ODC) inhibitor and a spermidine/spermine *N*¹-acetyltransferase expression agonist comprising assessing dietary polyamine intake by a patient to be treated with said combination therapy, wherein a high dietary polyamine intake level predicts a lower efficacy for said treatment.

As described herein, the agent that modulates the polyamine pathway to reduce overall cellular polyamine content is a spermidine/spermine *N*¹-acetyltransferase expression agonist. In some embodiments, said dietary polyamine level is determined from a patient dietary history.

In embodiment of the present invention, a high dietary polyamine level is defined as 300 µmol polyamine per day or higher, and said patient is administered said combination therapy if said dietary polyamine level is not high. The method can also further comprise subjecting said patient to a low polyamine diet prior to or at the time of commencing said combination therapy. As described herein, it can be useful to obtain results of a test that determines said patient's genotype at position +316 of at least one *ODC1* promoter gene allele. For example, said results may be obtained by receiving a report containing said genotype or taking a patient history that reveals said genotype.

In one aspect, the patient may be determined to possess a G at the nucleotide base at position +316 of at least one allele of the *ODC1* promoter gene of the patient; or wherein the patient's genotype at nucleotide at position +316 of both alleles of the *ODC1* promoter gene of the patient is GG or GA.

Also described herein there is provided method for treating a patient with carcinoma comprising:
a) assessing dietary polyamine intake by said patient and/or tissue polyamine levels or flux in said patient; and
b) administering to the patient a combined effective amount of a ornithine decarboxylase (ODC) inhibitor and an agent that modulates the polyamine pathway to reduce overall cellular polyamine content or flux.
The DFMO may be administered systemically. In some embodiments, sulindac or celecoxib are administered systemically. In one embodiment, the DFMO is administered orally. In one embodiment, the effective amount of DFMO is 500 mg/day. In embodiment, the DFMO is administered intravenously. In embodiment, the effective amount of DFMO is from about 0.05 to about 5.0 g/m²/day. Also described herein, the DFMO and the non-aspirin containing NSAID is formulated for oral administration. The DFMO and the non-aspirin containing NSAID may be formulated as a hard or soft capsule or a tablet. Also described herein, the DFMO and the non-aspirin containing NSAID is administered every 12 hours. The DFMO and the non-aspirin containing NSAID may be administered every 24 hours. In one embodiment, the effective amount of sulindac is from about 10 to about 1500 mg/day. Such as from about 10 to about 400 mg/day, or such as 150 mg/day. In one embodiment, DFMO is administered prior to sulindac. In one embodiment, DFMO is administered after sulindac. In embodiment, DFMO is administered before and after sulindac. In one embodiment, DFMO is administered concurrently with sulindac. In one embodiment, DFMO is administered at least a second time. In embodiment, sulindac is administered at least a second time. In one embodiment, said patient has a solid tumor, and said treatment further comprises resection of said solid tumor. In one embodiment, DFMO and sulindac are administered prior to said resection. In one embodiment, DFMO and sulindac are administered after said resection.

In some variations on any of the above embodiments, the carcinoma is colorectal cancer, breast cancer, pancreatic cancer, brain cancer, lung cancer, stomach cancer, a blood cancer, skin cancer, testicular cancer, prostate cancer, ovarian cancer, liver cancer or esophageal cancer, cervical cancer, head and neck cancer, non-melanoma skin cancer, neuroblastoma and glioblastoma. In one embodiment, the carcinoma is colorectal cancer. In some embodiments, the colorectal cancer is stage I. In some embodiments, the colorectal cancer is stage II. In some embodiments, the colorectal cancer is stage III. In some embodiments, the colorectal cancer is stage IV.

In another aspect the present invention relates to preventing recurrence of carcinoma in a patient having been previously diagnosed carcinoma.

In some embodiments of the present invention, said patient has previously had surgical resection of a carcinoma tumor.

Also described herein, there is provided method of rendering a carcinoma tumor in a patient resectable comprising:
a) assessing dietary polyamine intake by said patient and/or tissue polyamine levels in said patient; and
b) administering to the patient a combined effective amount of an ornithine decarboxylase (ODC) inhibitor and a spermidine/spermine *N*¹-acetyltransferase expression agonist.

The method can further comprise resecting said tumor from said patient following step b).

Also described herein, there is provided a method of preventing carcinoma in a patient having a familial history of carcinoma comprising:
a) assessing dietary polyamine intake by said patient, tissue polyamine levels or tissue polyamine flux in said patient; and
b) administering to the patient a combined effective amount of an ornithine decarboxylase (ODC) inhibitor and a spermidine/spermine *N*¹-acetyltransferase expression agonist.

In variations on any of the above embodiments, the patient is human.

The use of the word "a" or "an," when used in conjunction with the term "comprising" in the claims and/or the specification may mean "one," but it is also consistent with the meaning of "one or more," "at least one," and "one or more than one."

Throughout this application, the term "about" is used to indicate that a value includes the inherent variation of error for the device, the method being employed to determine the value, or the variation that exists among the study subjects.

The terms "comprise," "have" and "include" are open-ended linking verbs. Any forms or tenses of one or more of these verbs, such as "comprises," "comprising," "has," "having," "includes" and "including," are also open-ended. For example, any method that "comprises," "has" or "includes" one or more steps is not limited to possessing only those one or more steps and also covers other unlisted steps.

The term "effective," as that term is used in the specification and/or claims, means adequate to accomplish a desired, expected, or intended result.

As used herein, the term "IC₅₀" refers to an inhibitory dose which is 50% of the maximum response obtained.

As used herein, the term "patient" or "subject" refers to a living mammalian organism, such as a human, monkey, cow, sheep, goat, dog, cat, mouse, rat, guinea pig, or transgenic species thereof. In certain embodiments, the patient or subject is a primate. Nonlimiting examples of human subjects are adults, juveniles, infants and fetuses.

"Pharmaceutically acceptable" means that which is useful in preparing a pharmaceutical composition that is generally safe, non-toxic and neither biologically nor otherwise undesirable and includes that which is acceptable for veterinary use as well as human pharmaceutical use.

"Prevention" or "preventing" includes: (1) inhibiting the onset of a disease in a subject or patient which may be at risk and/or predisposed to the disease but does not yet experience or display any or all of the pathology or symptomatology of the disease, and/or (2) slowing the onset of the pathology or symptomatology of a disease in a subject or patient which may be at risk and/or predisposed to the disease but does not yet experience or display any or all of the pathology or symptomatology of the disease.

"Effective amount," "Therapeutically effective amount" or "pharmaceutically effective amount" means that amount which, when administered to a subject or patient for treating a disease, is sufficient to effect such treatment for the disease.

"Treatment" or "treating" includes (1) inhibiting a disease in a subject or patient experiencing or displaying the pathology or symptomatology of the disease (*e.g*., arresting further development of the pathology and/or symptomatology), (2) ameliorating a disease in a subject or patient that is experiencing or displaying the pathology or symptomatology of the disease (*e.g.,* reversing the pathology and/or symptomatology), and/or (3) effecting any measurable decrease in a disease in a subject or patient that is experiencing or displaying the pathology or symptomatology of the disease.

Other objects, features and advantages of the present disclosure will become apparent from the following detailed description.

### BRIEF DESCRIPTION OF THE DRAWINGS

The following drawings form part of the present specification and are included to further demonstrate certain aspects of the present disclosure. The invention may be better understood by reference to one of these drawings in combination with the detailed description of specific embodiments presented herein.

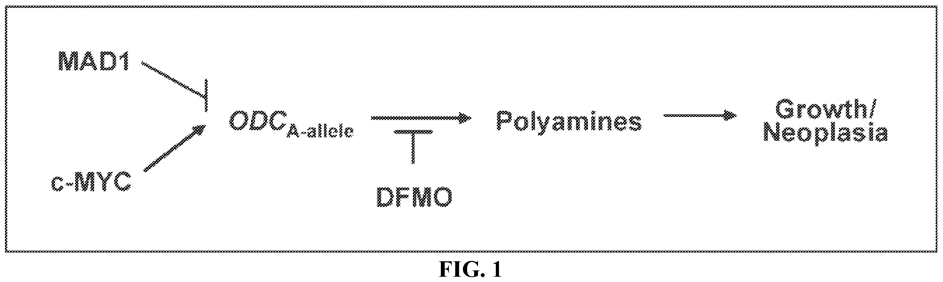

**FIG. 1** **- Differential Effects of Polyamine Regulation by *MAD1* and *c-MYC.*** Schema depicting the proposed differential effects of polyamine regulation by *MAD1* and *c-MYC* on the *ODC1* +316 minor A-allele. Effects of the ODC inhibitor DFMO (difluoromethylornithine) are also shown.

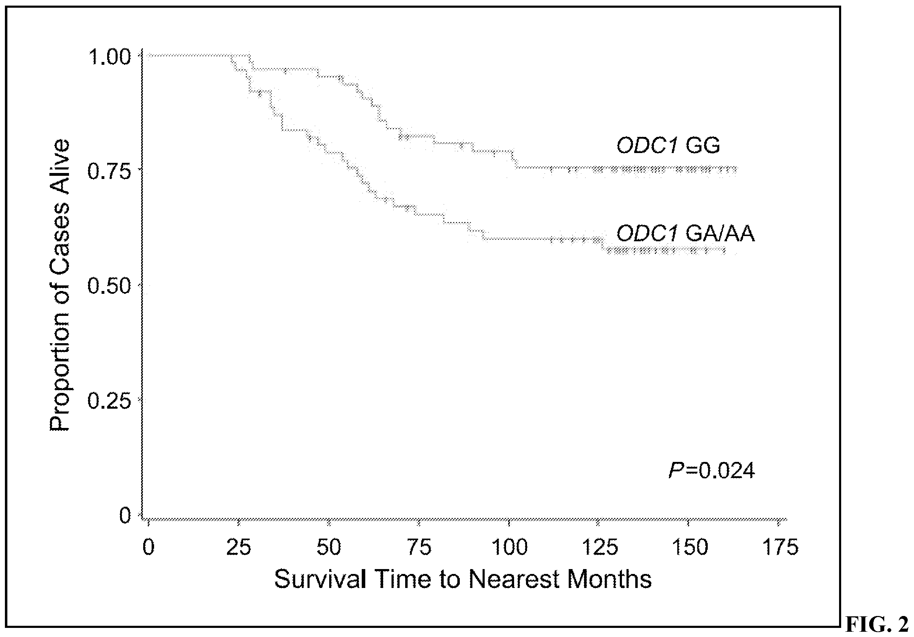

**FIG. 2** **- Colorectal Cancer-Specific Survival Rate Estimates.** This figure shows Kaplan-Meier colorectal cancer-specific survival rate estimates for cases with stage III colorectal cancer, stratified by *ODC1* +316 genotype. Included are cases from the University of California Irvine Gene-Environment Study of Familial Colorectal Cancer diagnosed during the period 1994-1996 with follow-up through March 2008: *ODC1* GG (64 cases, 15 colorectal cancer-specific deaths), *ODC1* GA/AA (62 cases, 25 colorectal cancer-specific deaths).

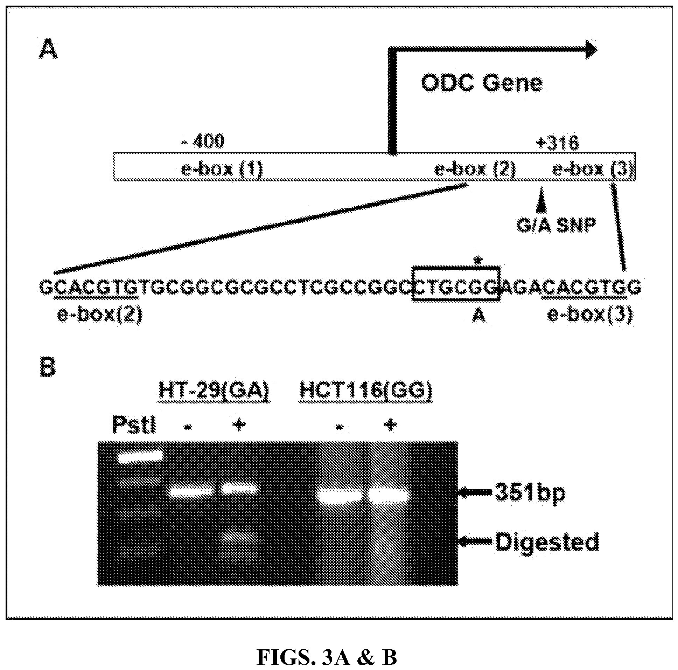

**FIGS. 3A** & **B** - **Location and Analysis of the *ODC1* promoter SNP.** FIG. A shows the A, location of the *ODC1* promoter SNP. The SNP under investigation in this study is 316 nucleotides 3' of the *ODC1* transcription start site (*). This SNP resides between two consensus E-boxes as shown by the underlined sequences, and affects a PstI restriction site (box) (SEQ ID NO:5). FIG. 3B shows a restriction fragment length polymorphism analysis of *ODC1* SNP. The DNA was obtained from two cell types, and the region surrounding the *ODC1* SNP site was sequenced. Colon-derived HT29 cells were found to be heterozygous GA, whereas HCT116 cells were found to be homozygous GG, at the *ODC1* SNP locus. A 350-bp PCR product of this region was obtained from each cell type and subjected to digestion with PstI. Evidence of an A-allele was indicated by restriction products <350 bp.

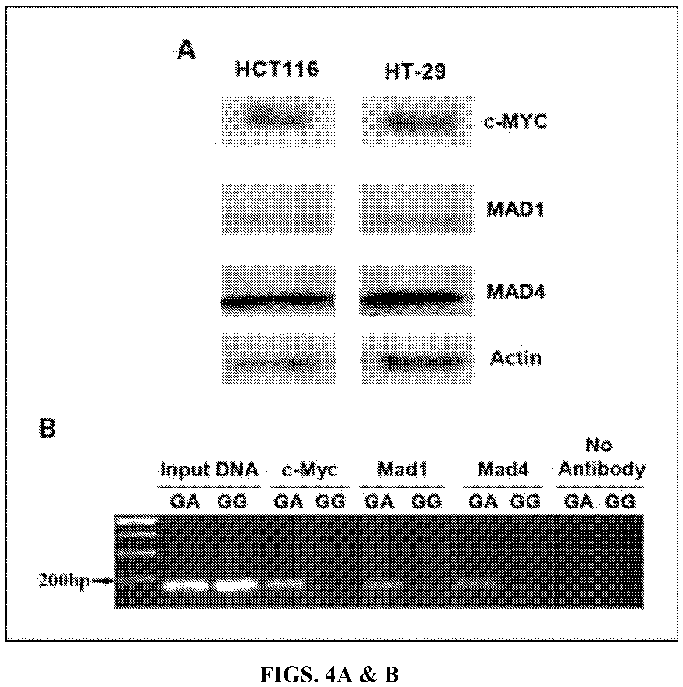

**FIGS. 4A & B** - **E-Box Expression and Immunoprecipitation Analysis. Location of the *ODC1* promoter SNP.** FIG. 4A shows E-box protein expression in colon-derived cells. Expression of proteins to be evaluated for binding to the +316 *ODC1* SNP was assessed by Western blot analysis. Extracts of both HT29 and HCT116 cells were evaluated for c-*MYC, MAD1*, and MAD4; β-actin was used as a loading control. FIG. 4B shows documentation for the allele-specific transcription factor binding by chromatin immunoprecipitation analysis, which was conducted as described in the examples section below. HT29 cells were a source of *ODC1* A-alleles, as these cells are heterozygous GA at this site. HCT116 cells were used as a source of *ODC1* G-alleles.

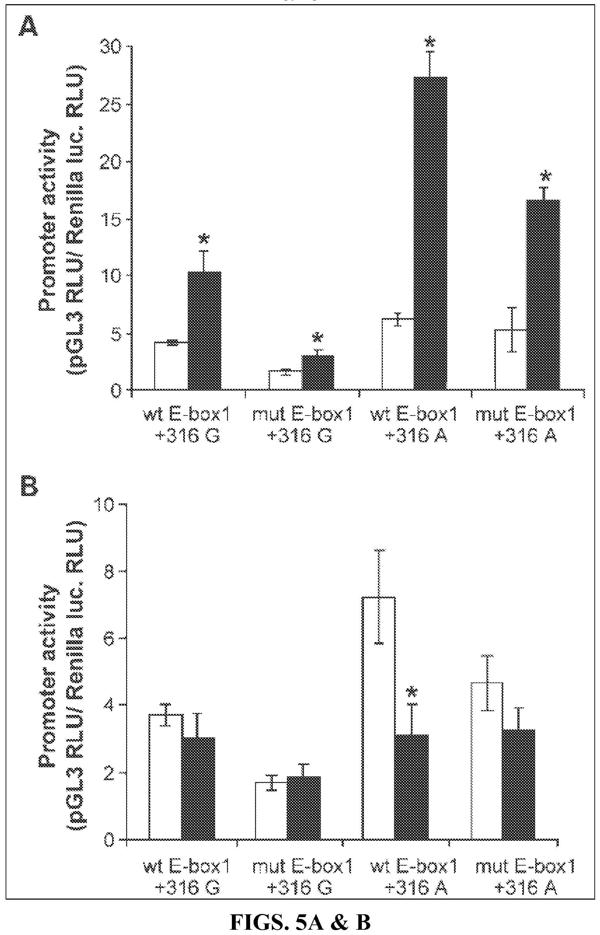

**FIGS. 5A & B** **- Effects of *c-MYC* and *MAD1* expression on ODC1 Activity.** FIG. 5A shows the effect of *c-MYC* expression on *ODC1* allele-specific promoter activity in HT29 colon-derived cells. Promoter activity was measured after transfection with *ODC1* promoter reporter plasmids co-transfected with pcDNA 3.0 plasmid or CMV-*MYC* expression vector. Promoter constructs differ by the presence of the first E-box element, located in -485 to -480 bp ("wt E-box1" for the wild-type sequence or "mut E-box1" for a mutant sequence). The constructs differ also by the *ODC1* +316 SNP ("+316 G" or "+316 A"). *, P ≤ 0.013 for each of the four comparisons relative to promoter activity with pcDNA 3.0 cotransfection. FIG. 5B shows the effect of *MAD1* expression on *ODC1* allele-specific promoter activity in HT29 colon tumor derived cells. Promoter activity was measured after transfection with ODC1 promoter reporter plasmids cotransfected with pcDNA 3.1 plasmid or with a pcDNA-*MAD1* plasmid. Promoter constructs used were described in the legend for panel A of this figure. *, P = 0.027, statistical significance relative to promoter activity with pcDNA 3.1 cotransfection.

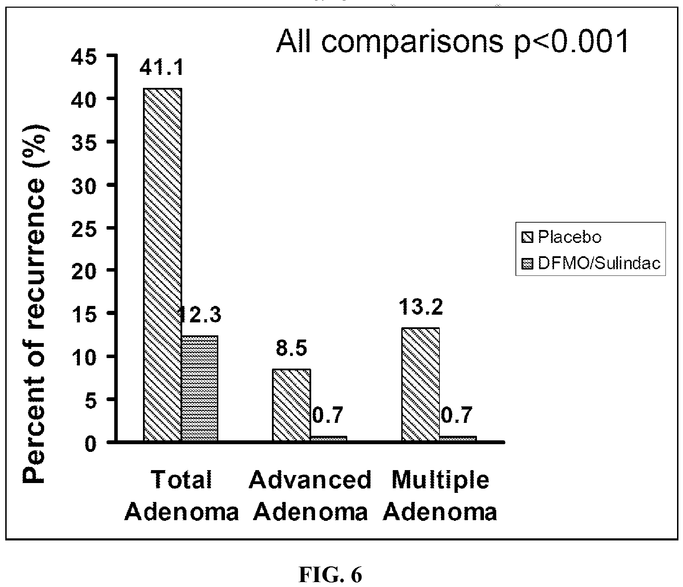

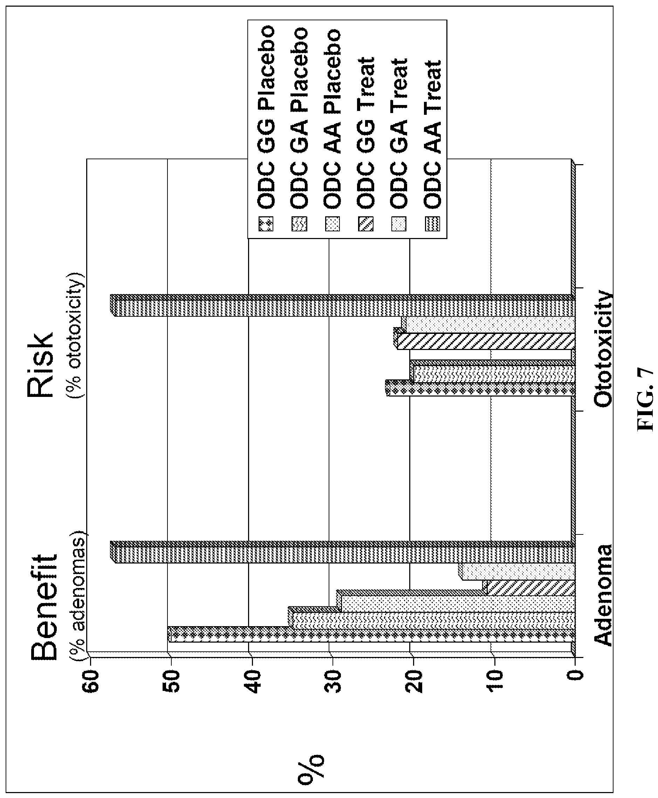

**FIG. 6** - **Reduction in Adenomatous Polyps.** This figure shows the percent recurrence of adenomatous polyps of patients were treated with DFMO and Sulindac compared with placebo. There was a 70% reduction in total adenoma, a 92% reduction in advanced adenoma, and 95% reduction in multiple adenoma.
**FIG**. 7 - **Pharmacogenomic Benefit/Risk Analysis Based on +316 *ODC1* Genotype.** This figure compares reduction in % recurrence of adenomas at the end of 3 years versus placebo, with % ototoxicity for treatment and placebo groups as a function of the patient's +316 *ODC1* genotype. Ototoxicity was determined using audiometric testing.

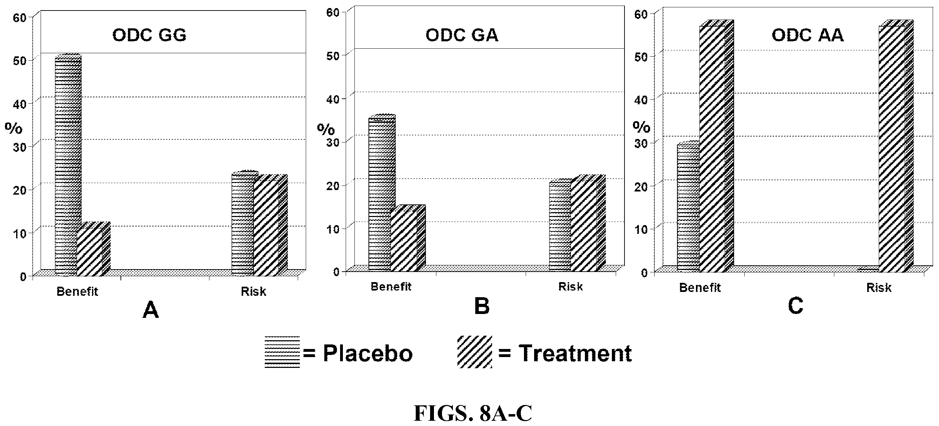

**FIG. 8A-C** - **Pharmacogenomic Benefit/Risk Analysis Based on +316 *ODC1* Genotype.** This figure compares benefit, reduction in % recurrence of adenomas at the end of 3 years, with risk, % ototoxicity, for treatment and placebo groups as a function of the patient's +316 *ODC1* genotype. Ototoxicity was determined using audiometric testing.

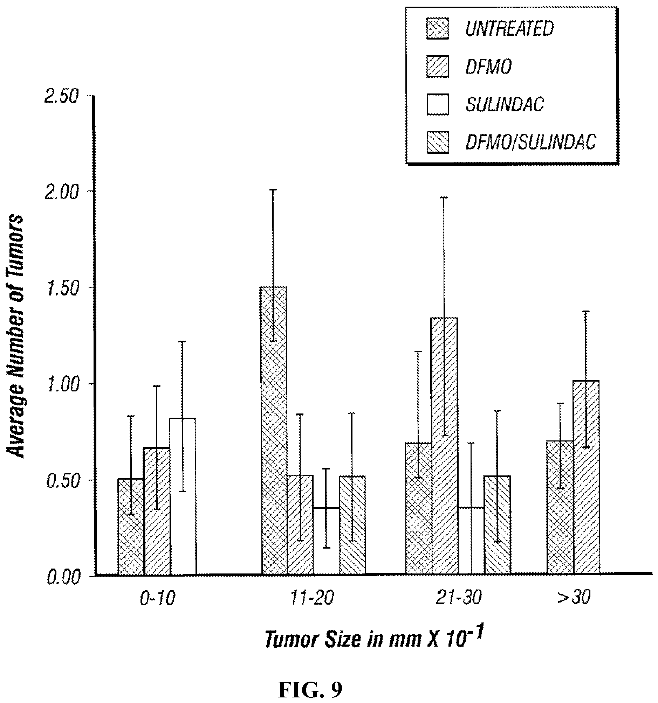

**FIG. 9** - **Average Number of Tumors by Size in Colon of *Minl+* Mice**. This figure shows the average number of tumors by size in the colon of the three treatment groups compared to untreated controls. Mice, purchased from The Jackson Laboratory (Bar Harbor, Me.), were bred crossing C57BL/6J- *Apc*^{*Min*/+} males and C57/BL6 females. Heterozygous Min mice (*Apc^{Min}lApc*⁺): (heterozygous for a nonsense mutation at codon 850 of *Apc*) were identified by genotyping at weaning by an allele specific PCR assay using tail-tip DNA. Homozygous (*Apc*⁺ /*Apc*⁺) litter mates served as controls. One treatment consisted of supplementing drinking water with 2% DFMO (Merrell Dow Research Inst.) on the 8th day of study. In the other treatment, 167 ppm of sulindac (Harlen Teklad) was added to AIN-93G mouse diet on the 21st day of the study. The third treatment was a combination of DFMO and sulindac. After 114 days, the mice were sacrificed through CO₂ asphyxiation. The small intestine and colon segments were removed from mice and dissected lengthwise, mounted and fixed in 70% ethanol, and placed at 4 °C for tumor scoring. Representative tissues were also taken for histopathology evaluation.
**FIG. 10** **- Average Number of Tumors by Size in the Small Intestine of *Minl*+ Mice.** This figure shows the average number of tumors by size in the small intestine of the three treatment groups compared to untreated controls. For experimental details, see FIG. 9 description above.
**FIG. 11** **- Number of High Grade Adenomas as a Function of Therapy in *Minl*+ Mice.** This figure shows how the number of high grade adenomas various depending on therapy type. For experimental details, see FIG. 9 description above.
**FIG. 12** **-Total Daily Dietary Polyamine and Total Daily Protein Intake.** Correlation at baseline between daily dietary total polyamine intake and intake of daily total protein from all sources. *P* value is reported using Spearman's rank correlation coefficient (*r*ₛ).
**FIG. 13** **-Total Daily Dietary Polyamine and Total Daily Arginine Intake.** Correlation at baseline between daily dietary total polyamine intake and intake of daily total arginine. *P* value is reported using Spearman's rank correlation coefficient (*rₛ*).

### DESCRIPTION OF ILLUSTRATIVE EMBODIMENTS

Controlling exogenous polyamines may be used, in some aspects, as an adjunctive strategy to chemoprevention with polyamine inhibitory agents, for example, anti-carcinoma combination therapies comprising ornithine decarboxylase (ODC) inhibitor and a spermidine/spermine *N*¹-acetyltransferase expression agonist, optionally based on a patient's *ODC1* promoter genotype. Assessing a tissue polyamine level or tissue polyamine flux may be used in some aspects, for predicting the efficacy of an anti-carcinoma combination therapy comprising, for example, an ornithine decarboxylase (ODC) inhibitor and an agent that modulates the polyamine pathway to reduce overall cellular polyamine content.

The present invention also involves in some aspects the delivery of therapeutic compounds to individuals exhibiting pre-cancerous symptoms to prevent the onset of cancer and/or to prevent the onset of cancer risk factors, such as the formation of new aberrant crypt foci, the formation of new adenomatous polyps or new adenomas with dysplasia. Cells of this category include polyps and other precancerous lesions, premalignancies, preneoplastic or other aberrant phenotype indicating probable progression to a cancerous state, based at least in part on the patient's *ODC1* promoter genotype.

### I. Polyamines Metabolism

Excess polyamine formation has long been implicated in epithelial carcinogenesis, particularly colorectal carcinogenesis. Polyamines are small ubiquitous molecules involved in various processes, including transcription, RNA stabilization, ion channel gating and others (Wallace, 2000). Ornithine decarboxylase (ODC), the first enzyme in polyamine synthesis, is essential for normal development and tissue repair in mammals but is down-regulated in most adult tissues (Gerner and Meyskens, 2004). Multiple abnormalities in the control of polyamine metabolism and transport result in increased polyamine levels that can promote tumorigenesis in several tissues (Thomas and Thomas, 2003).

Polyamine metabolism is up-regulated in intestinal epithelial tissues of humans with familial adenomatous polyposis (FAP) (Giardiello *et al*., 1997), a syndrome associated with high risk of colon and other cancers.

FAP may be caused by mutations in the adenomatous polyposis coli (*APC*) tumor suppressor gene, and APC signaling has been shown regulates ODC e*x*pression in both human cells (Fultz and Gerner, 2002) and in a mouse model of FAP (Erdman *et al*., 1999).

Wild type *APC* expression leads to decreased expression of ODC, while mutant APC leads to increased expression of ODC. The mechanism of APC-dependent regulation of ODC involves E-box transcription factors, including the transcriptional activator *c-MYC* and the transcriptional repressor *MAD1* (Fultz and Gerner, 2002; Martinez *et al*., 2003). *c-MYC* was shown by others to regulate ODC transcription (Bellofernandez *et al*., 1993). Several genes involved in polyamine metabolism are essential genes for optimal growth in most organisms, and are down-regulated in non-proliferating and/or adult cells and tissues (Gerner and Meyskens, 2004). The polyamines influence specific cellular phenotypes, in part, by affecting patterns of gene expression, as reviewed elsewhere (Childs *et al*., 2003).

As described below, a strategy involving inhibition of ODC activity (*i.e.,* the rate-limiting enzyme of polyamine synthesis) and/or reduction of cellular polyamine levels has demonstrated remarkable efficacy in preventing recurrence of colorectal polyps in humans. Epidemiologic and experimental results from the present research demonstrate conditional regulation of polyamine homeostasis by genetic polymorphism in ODC, and suggest a model in which the +316 ODC SNP may be protective for colon adenoma recurrence and detrimental for survival after colon cancer diagnosis. This information may be used for determining colon cancer prognosis. By identifying patients at increased risk for cancer progression/recurrence, early implementation of tertiary prevention management strategies can be instituted. Additionally, this research may be used to identify high-risk but otherwise optimally-treated locoregional colorectal cancer patients that would benefit from tertiary cancer prevention therapies.

Depending on a patient's diet, the excess polyamine problem may be compounded by the fact that polyamines, *e.g.*, putrescine is present in many common foods, such as orange juice, which contains approximately 400 ppm putrescine. In this regard, a high polyamine diet is contraindicatory, and for some of the embodiments provided herein such a diet is to be avoided.

### II. Assessing Dietary Polyamine Intake

Dietary intake of a subject can be accurately estimated by obtaining a patient history that records the daily average intake of various foodstuffs including those having significant polyamine content. For example, the Fred Hutchinson Cancer Research Center food frequency questionnaire (FFQ), and the analytic algorithms for this instrument can be utilized. Kristal AR, Shattock, A. L., and Williams, A. E.: Food frequency questionnaires for diet intervention research. Washington, DC: International Life Sciences Institute,. Proceeding of the 17th National Nutrient Databank Conference, Baltimore, MD, 1992., 1992; Schakel SF, Buzzard, I M., and Gebhardt, S. E. : Procedures for estimating nutrient values for food composition databases.. J Food Comp Anal 10: 102-14, 1997. To estimate dietary polyamine intake, the polyamine food content database developed and linked to the FFQ, for which the University of Minnesota Nutrition Coordinating Center (NCC) Nutrient Database serves as the primary source of food content data, may be used (Zoumas-Morse C, Rock CL, Quintana EL, et al: Development of a polyamine database for assessing dietary intake. J Am Diet Assoc 107:1024-7, 2007). Values for spermine, spermidine and putrescine in individual food items may be utilized, but alternatively, dietary putrescine may be assessed alone as the major contributor to total dietary polyamine intake. The results may also be energy-adjusted.

### III. Assessing Tissue Polyamine Levels

Tissue polyamine content can be determined from a biopsy using a variety of different tissue sources including rectal mucosal tissue, prostate tissue and urine. Preferably, the tissue will permit a relatively non-invasive biopsy process with minimal discomfort and recovery times. A particular source that has been utilized is the rectal mucosa, as described in the literature. Meyskens FL, Gerner EW, Emerson S, et al: Effect of alpha-difluoromethylornithine on Rectal Mucosal levels of polyamines in a randomized, double-blinded trial for colon cancer prevention. Journal of the National Cancer Institute 90:1212-1218, 1998; Boyle JO, Meyskens FL, Jr., Garewal HS, et al: Polyamine contents in rectal and buccal mucosae in humans treated with oral difluoromethylornithine. Cancer Epidemiol Biomarkers Prev 1:131-5, 1992. Samples may be flushed with ice-cold saline and stored frozen at -80°C until use.

Samples are be processed for the appropriate assay and assessed for polyamine content (putrescine, cadaverine, histamine, spermidine, spermine, and monoacetyl derivatives of putrescine, spermidine, and spermine) by acceptable methods, such as reverse-phase high performance liquid chromatography with 1,7-diaminoheptane as an internal standard. Meyskens FL, Gerner EW, Emerson S, et al: Effect of alpha-difluoromethylornithine on Rectal Mucosal levels of polyamines in a randomized, double-blinded trial for colon cancer prevention. Journal of the National Cancer Institute 90:1212-1218, 1998; Seiler N, Knodgen B: High-performance liquid chromatographic procedure for the simultaneous determination of the natural polyamines and their monoacetyl derivatives. J.Chromatogr. 221:227-235, 1980. Total protein content in samples
may be determined using techniques known in the art, such as those described in the commercially available BCA Protein Assay Kit (Pierce, Rockford, IL).

### IV. Low Polyamine Diet

When a subject is found to have high levels of tissue polyamines, or to have an unacceptably high polyamine dietary intake, it may be recommended that they reduce their polyamine intake prior to or as part of a therapeutic regimen involving an ornithine decarboxylase inhibitor and a spermidine/spermine *N*¹-acetyltransferase expression agonist in accordance with the present invention. Even subjects with only moderate tissue polyamine or dietary intake may benefit from a low polyamine dient.

Polyamine content of dietary foods varies widely, but in general, the following foods should be avoided when seeking to reduce polyamine intake: oranges, grapefruit, pears, bananas, cheeses, potatoes, pudding, cured meats, sausage, pork stew, cod, fried fish, anchovy, peas, beans, onion, tomato, red wine, nuts herbs, lentils. A reduction in at least some of these foods, and elimination of a few such as oranges, grapefruit, cheese and tomato, can have a significant impact on polyamine levels.

### V. Familial Adenomatous Polyposis

Familial Adenomatous Polyposis (FAP), an inherited polyposis syndrome, is the result of germ-line mutation of the adenomatous polyposis coli (APC) tumor suppressor gene (Su *et al*., 1992). This autosomal-dominant condition with variable expression is associated with the development of hundreds of colonic adenomas, which uniformly progress to adenocarcinoma by forty years of age, two decades earlier than the mean age diagnosis for sporadic colon cancer (Bussey, 1990). In prior studies of pre-symptomatic individuals with FAP, increased levels of the polyamines spermidine and spermine, and their diamine precursor putrescine, have been detected in normal-appearing colorectal biopsies when compared to normal family member controls (Giardiello *et al*., 1997). The activity of ornithine decarboxylase (ODC), the first and rate-limiting enzyme in mammalian polyamine synthesis, also is elevated in apparently normal colonic mucosal biopsies from FAP patients (Giardiello *et al*., 1997; Luk and Baylin, 1984). These findings are of interest as the polyamines are necessary for optimal cell proliferation (Pegg, 1986). Further, suppression of ODC activity, using the enzyme-activated irreversible inhibitor DFMO, inhibits colon carcinogenesis in carcinogen-treated rodents (Kingsnorth *et al*., 1983; Tempero *et al*., 1989).

As discussed in greater detail below, the Min (multiple intestinal neoplasia) mouse, which shares a mutated *APC*/*apc* genotype with FAP, serves as a useful experimental animal model for human FAP patients (Lipkin, 1997). The Min mouse can develop greater than 100 gastrointestinal adenomas/adenocarcinomas throughout the gastrointestinal tract by 120 days of life leading to GI bleeding, obstruction and death. A combination therapy of DFMO and sulindac was shown to be effective in reducing adenomas in these mice (US Patent No. 6,258,845; Gerner and Meyskens, 2004). The results of treating Min mice with either DFMO alone, sulindac alone, or a combination of DFMO and sulindac on tumor formation in either the colon or small intestine are shown in FIGS. 9-11.

### VI. Ornithine Decarboxylase-1 Polymorphism

Activity of ornithine decarboxylase (ODC), the first enzyme in polyamine synthesis, is required for normal growth and is elevated in many cancers, including colorectal cancer. Herein associations of the +316 ODC single nucleotide polymorphism (SNP) with colorectal cancer (CRC)-specific survival among CRC cases were examined and its functional significance in colon cancer cells was investigated.

A single nucleotide polymorphism (SNP) in intron-1 of the human *ODC1* gene affects *ODC1* transcription (Guo *et al*., 2000), and has been investigated as a genetic marker for colorectal adenoma (CRA) risk (Martinez *et al*., 2003; Barry *et al*., 2006; Hubner *et al*., 2008). The reported minor A-allele frequency is approximately 25% and despite differences across race/ethnicity, *ODC1* genotype distribution is in Hardy-Weinberg equilibrium within each race (O'Brien *et al*., 2004; Zell *et al*., 2009). Individuals homozygous for the *ODC1* minor A-allele have reduced risk of adenoma recurrence compared to those with the major G-allele (Martinez *et al*., 2003; Hubner *et al*., 2008). Furthermore, the *ODC1* A-allele (AA or GA genotype, but not GG genotype) and reported aspirin usage have been associated with reduced colon polyp recurrence (Martinez *et al*., 2003; Barry *et al*., 2006; Hubner *et al*., 2008), and a statistically significant 50% reduced risk of advanced adenomas (Barry *et al*., 2006).

The ODC allele-specific binding of E-box transcription factors was investigated and the functional significance of the +316 ODC SNP, located between two E-boxes was evaluated (E-box 2 and 3 as depicted in FIG. 2A). Each cell line genotype influences a consensus PstI restriction site in this region. FIG. 2B shows that a polymerase chain reaction (PCR) product made from human colon HT29 cells was partially sensitive to PstI cutting, suggesting that these cells contained at least one ODC A-allele. A PCR product made from human colon HCT116 cells using the same primers was insensitive to PstI action, implying that these cells contained only ODC G-alleles. This result was confirmed by direct DNA sequencing.

Expression of specific E-box binding proteins, including the transcriptional activator *c-MYC* and several transcriptional repressors in HT29 and HCT116 cells (*e.g. MAD1* and MAD4), was established by Western blotting (FIG. 3A). Chromatin immunoprecipitation (CHIP) analysis of the region surrounding +316 of the ODC promoter was conducted, using antibodies directed against these proteins. As shown in FIG. 3B, ODC promoter-specific PCR products were synthesized from HT29 DNA obtained after immunoprecipitation of chromatin with antibodies directed against *c-MYC*, *MAD1* or MAD4. PCR products synthesized from HCT116 DNA after similar chromatin immunoprecipitation were substantially reduced compared to those synthesized from HT29 DNA. Quantification of these results indicated that *c-MYC*, *MAD1*, and MAD4 binding to the ODC SNP region was 4-14 times greater in HT29 cells, which contained one ODC-A allele, compared to HCT116 cells, which contained only ODC-G alleles.

ODC allele-specific promoter activity was assessed. The hypothesis that +316 ODC SNP influenced ODC expression in a manner dependant on the expression of E-box activators and repressors was tested as follows. Transient co-transfection of colon cancer-derived HT29 cells was accomplished with ODC allele-specific promoter constructs in combination with vectors expressing either the transcriptional activator *c-MYC* or the repressor *MAD1* (FIGS. 4A & B). The standard error bars shown reflect the variability in triplicate measurements within a single representative experiment, which has been replicated. The allele-specific promoter-reporters used in these experiments included all three E-boxes shown in FIG. 2A. As shown in FIG. 4A, *c-MYC* expression had the greatest stimulatory effect on promoters containing three consensus E-boxes and the ODC-A allele (wt E-box1 +316 A, P = 0.0014). Deletion of the upstream E-box reduced promoter activity, but *c-MYC* expression continued to stimulate this activity (mut E-box1 +316 A, P = 0.0013). Substitution of a G for the A at the +316 SNP position reduced the ability of *c-MYC* to stimulate promoter activity even with an intact 5' flanking consensus E-box. Mutation of the 5' flanking consensus E-box in combination with the ODC-G allele further reduced promoter activity.

When *MAD1*, rather than *c-MYC,* was co-transfected with the ODC allele-specific promoter reporters (FIG. 4B), the repressor was only able to reduce the activity of the ODC promoter which contained all three E-boxes and the wild-type +316 A-allele (P = 0.027). Deletion of the upstream E-box (mut E-box 1 +316A) significantly reduced the effect of *MAD1* on ODC promoter activity. Substitution of G for A at the +316 position rendered promoters containing either two or three E-boxes unresponsive to *MAD1* suppression.

### VII. Difluoromethylornithine (DFMO)

DFMO, also know as eflornithine, has the following chemical designation; 2-(difluoromethyl)-*dl*-ornithine. It is an enzyme-activated irreversible inhibitor of ornithine decarboxylase (ODC), the rate limiting enzyme of the polyamine biosynthetic pathway. As a result of this inhibition of polyamine synthesis, the compound is effective in preventing cancer formation in many organ systems, inhibiting cancer growth, and reducing tumor size. It also has synergistic action with other antineoplastic agents.

DFMO has been shown to decrease APC-dependent intestinal tumorigenesis in mice (Erdman *et al*., 1999). Oral DFMO administered daily to humans inhibits ODC enzyme activity and polyamine contents in a number of epithelial tissues (Love *et al*., 1993; Gerner *et al*., 1994; Meyskens *et al*., 1994; Meyskens *et al*., 1998; Simoneau *et al*., 2001; Simoneau *et al*., 2008). Recently, the inventors reported that DFMO in combination with the non-steroidal anti-inflammatory drug (NSAID) sulindac, has been reported to markedly lower the adenoma recurrence rate among individuals with colonic adenomas when compared to placebos in a randomized clinical trial (Meyskens *et al*., 2008).

DFMO was originally synthesized by Centre de Recherche Merrell, Strasbourg; Current FDA approvals include
- African sleeping sickness. High dose systemic IV dosage form-not marketed (Sanofi/WHO)
- Hirsutis (androgen-induced excess hair growth) topical dosage form

No oral formulations are currently approved.

DFMO and its use in the treatment of benign prostatic hypertrophy are described in two patents, U.S. Pat. Nos. 4,413,141, and 4,330,559. U.S. Pat. No. 4,413,141 describes DFMO as being a powerful inhibitor of ODC, both *in vitro* and *in vivo.* Administration of DFMO causes a decrease in putrescine and spermidine concentrations in cells in which these polyamines are normally actively produced. Additionally, DFMO has been shown to be capable of slowing neoplastic cell proliferation when tested in standard tumor models. U.S. Pat. No. 4,330,559 describes the use of DFMO and DFMO derivatives for the treatment of benign prostatic hypertrophy. Benign prostatic hypertrophy, like many disease states characterized by rapid cell proliferation, is accompanied by abnormal elevation of polyamine concentrations. The treatment described within this reference can be administered to a patient either orally, or parenterally.

DFMO can potentially be given continuously with significant anti-tumor effects. This drug is relatively non-toxic at low doses of 0.4 g/m²/day to humans while producing inhibition of putrescine synthesis in tumors. Studies in a rat-tumor model demonstrate that DFMO infusion can produce a 90% decrease in tumor putrescine levels without suppressing peripheral platelet counts.

Side effects observed with DFMO include effects on hearing at high doses of 4 g/M²/day that resolve when it is discontinued. These effects on hearing are not observed at lower doses of 0.4g/M²/day when administered for up to one year (Meyskens *et al*., 1994). In addition a few cases of dizziness/vertigo are seen that resolve when the drug is stopped. Thrombocytopenia has been reported predominantly in studies using high "therapeutic" doses of DFMO (>1.0 g/m²/day) and primarily in cancer patients who had previously undergone chemotherapy or patients with compromised bone marrow. Although the toxicity associated with DFMO therapy are not, in general, as severe as other types of chemotherapy, in limited clinical trials it has been found to promote a dose-related thrombocytopenia. Moreover, studies in rats have shown that continuous infusion of DFMO for 12 days significantly reduces platelet counts compared with controls. Other investigations have made similar observations in which thrombocytopenia is the major toxicity of continuous i.v. DFMO therapy. These findings suggest that DFMO may significantly inhibit ODC activity of the bone marrow precursors of megakaryocytes. DFMO may inhibit proliferative repair processes, such as epithelial wound healing.

A phase III clinical trial assessed the recurrence of adenomatous polyps after treatment for 36 months with difluoromethylornithine (DFMO) plus sulindac or matched placebos. Temporary hearing loss is a known toxicity of treatment with DFMO, thus a comprehensive approach was developed to analyze serial air conduction audiograms. The generalized estimating equation method estimated the mean difference between treatment arms with regard to change in air conduction pure tone thresholds while accounting for within-subject correlation due to repeated measurements at frequencies. Based on 290 subjects, there was an average difference of 0.50 dB between subjects treated with DFMO plus sulindac compared with those treated with placebo (95% confidence interval, -0.64 to 1.63 dB; P = 0.39), adjusted for baseline values, age, and frequencies. In the normal speech range of 500 to 3,000 Hz, an estimated difference of 0.99 dB (-0.17 to 2.14 dB; P = 0.09) was detected. Dose intensity did not add information to models. There were 14 of 151 (9.3%) in the DFMO plus sulindac group and 4 of 139 (2.9%) in the placebo group who experienced at least 15 dB hearing reduction from baseline in 2 or more consecutive frequencies across the entire range tested (P = 0.02). Follow-up air conduction done at least 6 months after end of treatment showed an adjusted mean difference in hearing thresholds of 1.08 dB (-0.81 to 2.96 dB; P = 0.26) between treatment arms. There was no significant difference in the proportion of subjects in the DFMO plus sulindac group who experienced clinically significant hearing loss compared with the placebo group. The estimated attributable risk of ototoxicity from exposure to the drug is 8.4% (95% confidence interval, -2.0% to 18.8%; P = 0.12). There is a <2 dB difference in mean threshold for patients treated with DFMO plus sulindac compared with those treated with placebo. The results of this study are discussed in greater detail in McLaren *et al*., 2008. Provided herein are methods of reducing and/or preventing ototoxicity in patients treated with agents such as DFMO and sulindac.

### VIII. NSAIDs

NSAIDs are anti-inflammatory agents that are not steroids. In addition to anti-inflammatory actions, they have analgesic, antipyretic, and platelet-inhibitory actions. They are used primarily in the treatment of chronic arthritic conditions and certain soft tissue disorders associated with pain and inflammation. They act by blocking the synthesis of prostaglandins by inhibiting cyclooxygenase, which converts arachidonic acid to cyclic endoperoxides, precursors of prostaglandins. Inhibition of prostaglandin synthesis accounts for their analgesic, antipyretic, and platelet-inhibitory actions; other mechanisms may contribute to their anti-inflammatory effects. Certain NSAIDs also may inhibit lipoxygenase enzymes or phospholipase C or may modulate T-cell function. (AMA Drug Evaluations Annual, 1814-5, 1994).

The nonsteroidal anti-inflammatory drugs (NSAIDs), including aspirin, ibuprofen, piroxicam (Reddy *et al*., 1990; Singh *et al*., 1994), indomethacin (Narisawa, 1981), and sulindac (Piazza *et al*., 1997; Rao *et al*., 1995), effectively inhibit colon carcinogenesis in the AOM-treated rat model. NSAIDs also inhibit the development of tumors harboring an activated Ki-ras (Singh and Reddy, 1995). NSAIDs appear to inhibit carcinogenesis via the induction of apoptosis in tumor cells (Bedi *et al*., 1995; Lupulescu, 1996; Piazza *et al*., 1995; Piazza *et al*., 1997b). A number of studies suggest that the chemopreventive properties of the NSAIDs, including the induction of apoptosis, is a function of their ability to inhibit prostaglandin synthesis (reviewed in DuBois *et al*., 1996; Lupulescu, 1996; Vane and Botting, 1997). Studies, however, indicate that NSAIDs may act through both prostaglandin-dependent and -independent mechanisms (Alberts *et al*., 1995; Piazza *et al*., 1997a; Thompson *et al*., 1995; Hanif, 1996). Sulindac sulfone, a metabolite of the NSAID sulindac, lacks COX-inhibitory activity yet induces apoptosis in tumor cells (Piazza *et al*., 1995; Piazza *et al*., 1997b) and inhibits tumor development in several rodent models of carcinogenesis (Thompson *et al*., 1995; Piazza *et al.,* 1995, 1997a).

Several NSAIDs have been examined for their effects in human clinical trials. A phase IIa trial (one month) of ibuprofen was completed and even at the dose of 300 mg/day, a significant decrease in prostoglandin E₂ (PGE₂) levels in flat mucosa was seen. A dose of 300 mg of ibuprofen is very low (therapeutic doses range from 1200-3000 mg/day or more), and toxicity is unlikely to be seen, even over the long-term. However, in animal chemoprevention models, ibuprofen is less effective than other NSAIDs.

### A. Sulindac and Its Major Metabolites, Sulindac Sulfone and Sulindac Sulfide

Sulindac is a non-steroidal, anti-inflammatory indene derivative with the following chemical designation; (*Z*)-5-fluoro-2-methyl-1-((4(methylsulfinyl)phenyl)methylene) 1*H-*indene-3-acetic acid (Physician's Desk Reference, 1999). The sulfinyl moiety is converted *in vivo* by reversible reduction to a sulfide metabolite and by irreversible oxidation to a sulfone metabolite (exisulind). See U.S. Patent 6,258,845. Sulindac, which also inhibits Ki-ras activation, is metabolized to two different molecules which differ in their ability to inhibit COX, yet both are able to exert chemopreventive effects via the induction of apoptosis. Sulindac sulfone lacks COX-inhibitory activity, and most likely facilitates the induction of apoptosis in a manner independent of prostaglandin synthesis. Available evidence indicates that the sulfide derivative is at least one of the biologically active compounds. Based on this, sulindac may be considered a prodrug.

Sulindac (Clinoril®) is available, for example, as 150 mg and 200 mg tablets. The most common dosage for adults is 150 to 200 mg twice a day, with a maximal daily dose of 400 mg. After oral administration, about 90% of the drug is absorbed. Peak plasma levels are achieved in about 2 hours in fasting patients and 3 to 4 hours when administered with food. The mean half-life of sulindac is 7.8 hours: the mean half-life of the sulfide metabolite is 16.4 hours. U.S. Pat. Nos. 3,647,858 and 3,654,349 cover preparations of sulindac.

Sulindac is indicated for the acute and long-term relief of signs and symptoms of osteoarthritis, rheumatoid arthritis, ankylosing spondylitis, acute gout, and acute painful shoulder. The analgesic and antiinflammatory effects exerted by sulindac (400 mg per day) are comparable to those achieved by aspirin (4 g per day), ibuprofen (1200 mg per day), indometacin (125 mg per day), and phenylbutazone (400 to 600 mg per day). Side effects of sulindac include mild gastrointestinal effects in nearly 20% of patients, with abdominal pain and nausea being the most frequent complaints. CNS side effects are seen in up to 10% of patients, with drowsiness, headache, and nervousness being those most frequently reported. Skin rash and pruritus occur in 5% of patients. Chronic treatment with sulindac can lead to serious gastrointestinal toxicity such as bleeding, ulceration, and perforation.

The potential use of sulindac for chemoprevention of cancers, and in particular colorectal polyps, has been well studied. Two recent U.S. Pat. Nos. 5,814,625 and 5,843,929, detail potential chemopreventive uses of sulindac in humans. Doses of sulindac claimed in U.S. Pat. No. 5,814,625 range from 10 mg to 1500 mg per day, with preferred doses of 50 mg to 500 mg per day. However, at the higher doses, the biggest problem with the use of sulindac as a single agent in chemoprevention is its well-known toxicities and moderately high risk of intolerance. The elderly appear to be especially vulnerable, as the incidence of side effects is higher in those over the age of 60. It is noted that this age group is most likely to develop colorectal cancer, and therefore, most likely to benefit from chemoprevention.

Sulindac and its sulfone metabolite exisulind have been tested and continue to be tested clinically for the prevention and treatment of several cancer types. Clinical Trials.gov, a U.S. National Institutes of Health database provides the following overview of as of May 10, 2010.

| **Status** | **Clinical Trial** | | |
|---|---|---|---|
| Recruiting | A Randomized Study of Sulindac in Oral Premalignant Lesions | | |
| | Conditions: | | Leukoplakia, Oral; Benign Neoplasms |
| | Interventions: | | Drug: sulindac; Drug: Placebo |
| | Sponsors: | | Memorial Sloan-Kettering Cancer Center; Head and Neck Surgery, AIMS, Cochin, India; Weill Medical College of Cornell University; Regional Cancer Centre (RCC), Trivandrum, India; Mazumdar Shaw Cancer Center (MSCC) |
| | Phase: | | Not listed |
| Recruiting | Sulindac in Preventing Melanoma in Healthy Participants Who Are at Increased Risk of Melanoma | | |
| | Condition: | | Precancerous Condition |
| | Interventions: | | Drug: sulindac; Other: placebo |
| | Sponsors: | | University of Arizona; |
| | Phase: | | National Cancer Institute (NCI) |
| | | | Phase II |
| Active, not recruiting | Eflornithine and Sulindac in Preventing Colorectal Cancer in Patients With Colon Polyps | | |
| | Conditions: | | Colorectal Cancer; Precancerous/Nonmalignant Condition |
| | Intervention: | | Drug: eflornithine plus sulindac |
| | Sponsors: | | University of California, Irvine; |
| | | | Chao Family Comprehensive Cancer Center; |
| | | | National Cancer Institute (NCI) |
| | Phase: | | Phase III |
| Completed | Sulindac in Preventing Breast Cancer in Women at High Risk for Breast Cancer | | |
| | Condition: | | Breast Cancer |
| | Interventions: | | Drug: sulindac; Other: laboratory biomarker analysis |
| | Sponsors: | | University of Arizona; |
| | Phase: | | National Cancer Institute (NCI) |
| | | | Phase I |
| Completed | Sulindac Capsules Compared With Sulindac Tablets in Healthy Volunteers | | |
| | Condition: | Unspecified Adult Solid Tumor, Protocol Specific | |
| | Interventions: | Drug: sulindac; Other: pharmacological study | |
| | Sponsors: | Mayo Clinic; National Cancer Institute (NCI) | |
| | Phase: | | |
| Active, not recruiting | Eflornithine Plus Sulindac in Preventing Colorectal Cancer in Patients With Benign Colorectal Polyps | | |
| | Condition: | | Colorectal Cancer |
| | Intervention: | | Drug: eflornithine plus sulindac |
| | Sponsors: | | University of California, Irvine; |
| | | | Chao Family Comprehensive Cancer Center; |
| | Phase: | | National Cancer Institute (NCI) |
| | | | Phase II |
| Active, not recruiting | Bevacizumab/Tarceva and Tarceva/Sulindac in Squamous Cell Carcinoma of the Head and Neck | | |
| | Condition: | | Squamous Cell Carcinoma of the Head and Neck (SCCHN) |
| | Interventions: | | Drug: Bevacizumab; Drug: erlotinib; |
| | | | Drug: Sulindac |
| | Sponsors: | | Massachusetts General Hospital; Dana-Farber Cancer Institute; Emory University; |
| | | | University of North Carolina, Chapel Hill; |
| | | | Genentech; OSI Pharmaceuticals |
| | Phase: | | Phase II |
| Active, not recruiting | Sulindac in Preventing Lung Cancer in Current or Former Smokers With Bronchial Dysplasia | | |
| | Conditions: | | Lung Cancer; Precancerous Condition; |
| | | | Tobacco Use Disorder |
| | Interventions: | | Drug: sulindac; Other: placebo |
| | Sponsors: | | Mayo Clinic; |
| | Phase: | | National Cancer Institute (NCI) |
| | | | Phase II |
| Completed | Sulindac and Tamoxifen in Treating Patients With Desmoid Tumor | | |
| | Condition: | | Desmoid Tumor |
| | Interventions: | | Drug: sulindac; Drug: tamoxifen citrate |
| | Sponsors: | | Children's Oncology Group; |
| | | | National Cancer Institute (NCI) |
| | Phase: | | Phase II |
| Recruiting | Sulindac and Epirubicin in Treating Patients With Metastatic Malignant Melanoma | | |
| | Condition: | Melanoma (Skin) | |
| | Interventions: | Drug: epirubicin hydrochloride; Drug: sulindac; | |
| | | Other: immunologic technique | |
| | Sponsor: | All Ireland Cooperative Oncology Research Group | |
| | Phase: | Phase II | |
| Completed | Atorvastatin, Oligofructose-Enriched Inulin, or Sulindac in Preventing Cancer in Patients at Increased Risk of Developing Colorectal Neoplasia | | |
| | Conditions: | | Colorectal Cancer; Precancerous Condition |
| | Interventions: | | Dietary Supplement: oligofructose-enriched inulin; Drug: atorvastatin calcium; |
| | | | Drug: sulindac; Other: placebo |
| | Sponsors: | | Mayo Clinic; |
| | | | National Cancer Institute (NCI) |
| | Phase: | | Phase II |
| Suspended | Sulindac and Plant Compounds in Preventing Colon Cancer | | |
| | Condition: | | Colorectal Cancer |
| | Interventions: | | Dietary Supplement: curcumin; |
| | | | Dietary Supplement: rutin; Drug: quercetin; |
| | | | Drug: sulindac |
| | Sponsor: | | Rockefeller University |
| | Phase: | | |
| Active, not recruiting | Comparison of Sulindac, Aspirin, and Ursodiol in Preventing Colorectal Cancer | | |
| | Condition: | | Colorectal Cancer |
| | Interventions: | | Drug: acetylsalicylic acid; Drug: sulindac; |
| | | | Drug: ursodiol |
| | Sponsors: | | M.D. Anderson Cancer Center; |
| | | | National Cancer Institute (NCI) |
| | Phase: | | Phase II |
| Completed | Sulindac and Docetaxel in Treating Women With Metastatic or Recurrent Breast Cancer | | |
| | Condition: | | Breast Cancer |
| | Interventions: | | Drug: docetaxel; Drug: sulindac |
| | Sponsors: | | Fox Chase Cancer Center; |
| | | | National Cancer Institute (NCI) |
| | Phase: | | Phase II |
| Recruiting | Influence of Sulindac and Probiotics on the Development of Pouch Adenomas in Patients With Familial Adenomatous Polyposis | | |
| | Condition: | | Adenomatous Polyposis Coli |
| | Interventions: | | Drug: Sulindac (drug); |
| | | | Drug: VSL#3 (probiotic); |
| | | | Drug: Inulin (probiotic) |
| | Sponsors: | | Radboud University; Dutch Cancer Society |
| | Phase: | | Phase II |
| Terminated | The Effects of Curcuminoids on Aberrant Crypt Foci in the Human Colon | | |
| | Condition: | Aberrant Crypt Foci | |
| | Interventions: | Drug: sulindac; Drug: curcumin | |
| | Sponsor: | University of Medicine and Dentistry New Jersey | |
| | Phase: | | |
| Recruiting | | Use of Curcumin for Treatment of Intestinal Adenomas in Familial Adenomatous Polyposis (FAP) | |
| | | Conditions: | Lower Tract Polyps in Patients With FAP; |
| | | | Upper Tract Polyps in Patients With FAP |
| | | Interventions: | Drug: Calcumin (Curcumin); |
| | | | Other: Risk Factor Questionnaire; |
| | | | Other: Blood samples; |
| | | | Other: Biopsies (Sigmoidoscopy); |
| | | | Other: Biopsies (Upper endoscopy) |
| | | Sponsor: | University of Puerto Rico |
| | | Phase: | |
| Active, not recruiting | | To Lengthen the Duration of the Off-Treatment of Intermittent Androgen Suppression | |
| | | Condition: | Prostate Cancer |
| | | Interventions: | Drug: Flutamide; |
| | | | Drug: Leuprolide Acetate; Drug: Exisulind |
| | | Sponsors: | University of Washington; |
| | | | OSI Pharmaceuticals |
| | | Phase: | Phase II |
| Completed | | Safety, Efficacy and Pharmacokinetic Between Capecitabine and Exisulind in Metastatic Breast Cancer Patients | |
| | | Conditions: | Breast Neoplasms; Metastases, Neoplasm |
| | | Interventions: | Drug: Capecitabine; Drug: Exisulind |
| | | Sponsors: | M.D. Anderson Cancer Center; |
| | | | Cell Pathways |
| | | Phases: | Phase I / Phase II |
| Completed | | Neoadjuvant Exisulind in Treating Patients Who Are Undergoing Radical Prostatectomy for Stage II or Stage III Prostate Cancer | |
| | | Condition: | Prostate Cancer |
| | | Interventions: | Drug: exisulind; |
| | | | Procedure: conventional surgery; |
| | | | Procedure: neoadjuvant therapy |
| | | Sponsors: | Mayo Clinic; |
| | | | National Cancer Institute (NCI) |
| | | Phase: | Phase II |
| Completed | | Combination Chemotherapy in Treating Patients With Advanced Non-Small Cell Lung Cancer | |
| | | Condition: | Lung Cancer |
| | | Interventions: | Drug: carboplatin; Drug: exisulind; |
| | | | Drug: gemcitabine hydrochloride |
| | | Sponsors: | Eastern Cooperative Oncology Group; |
| | | | National Cancer Institute (NCI) |
| | | Phase: | Phase II |

| **Status Clinical Trial** | | | |
|---|---|---|---|
| Completed | | Phase II Study of Taxotere in Combination With Exisulind in Non-Small Cell Lung Cancer (NSCLC) Patients | |
| | | Condition: | NSCLC |
| | | Intervention: | Drug: Exisulind |
| | | Sponsor: | OSI Pharmaceuticals |
| | | Phases: | Phase I / Phase II |
| Completed | | A Phase III Study of the Efficacy of Taxotere/Aptosvn Versus Taxotere/Placebo in Non-Small Cell Lung Cancer Patients | |
| | | Condition: | Non-Small Cell Lung Cancer |
| | | Intervention: | Drug: Exisulind |
| | | Sponsor: | OSI Pharmaceuticals |
| | | Phase: | Phase III |
| Completed | | Exisulind Versus Placebo After Surgical Removal of the Prostate | |
| | | Condition: | Prostatic Neoplasms |
| | | Intervention: | Drug: Exisulind |
| | | Sponsors: | Mayo Clinic; OSI Pharmaceuticals |
| | | Phase: | Phase II |
| Completed | | Docetaxel. Estramustine. and Exisulind in Treating Patients With Metastatic Prostate Cancer That Has Not Responded to Hormone Therapv | |
| | | Condition: | Prostate Cancer |
| | | Interventions: | Drug: docetaxel; |
| | | | Drug: estramustine phosphate sodium; |
| | | | Drug: exisulind |
| | | Sponsors: | Cancer and Leukemia Group B; |
| | | | National Cancer Institute (NCI) |
| | | Phase: | Phase II |
| Completed | | Combination Chemotherapy and Exisulind in Treating Patients With Extensive-Stage Small Cell Lung Cancer | |
| | | Condition: | Lung Cancer |
| | | Interventions: | Drug: carboplatin; Drug: etoposide; |
| | | | Drug: exisulind |
| | | Sponsors: | Cancer and Leukemia Group B; |
| | | | National Cancer Institute (NCI) |
| | | Phase: | Phase II |
| Active, not recruiting | | Exisulind in Preventing Polvps in Patients With Familial Adenomatous Polyposis | |
| | | Conditions: | Colorectal Cancer; Small Intestine Cancer |
| | | Intervention: | Drug: exisulind |
| | | Sponsor: | University of Utah |
| | | Phases: | Phase II / Phase III |

| **Status** | **Clinical Trial** | | |
|---|---|---|---|
| Completed | Exisulind Prior to Radical Prostatectomy | | |
| | Condition: | | Prostatic Neoplasms |
| | Intervention: | | Drug: Exisulind Therapy |
| | Sponsors: | | Mayo Clinic; |
| | | | National Cancer Institute (NCI) |
| | Phase: | | Phase II |

### B. Piroxicam

A non-steroidal anti-inflammatory agent that is well established in the treatment of rheumatoid arthritis and osteoarthritis with the following chemical designation; 4-hydroxy-2-methyl-*N*-2-pyridyl-2*H*-1,2-benzothiazine-3-carboxamide 1,1-dioxide. Its usefulness also has been demonstrated in the treatment of musculoskeletal disorders, dysmenorrhea, and postoperative pain. Its long half-life enables it to be administered once daily. The drug has been shown to be effective if administered rectally. Gastrointestinal complaints are the most frequently reported side effects.

Piroxicam has been shown to be effective chemoprevention agent in animal models (Pollard and Luckert, 1989; Reddy *et al*., 1987), although it demonstrated side effects in a recent IIb trial. A large meta-analysis of the side effects of the NSAIDs also indicates that piroxicam has more side effects than other NSAIDs (Lanza *et al*., 1995). Sulindac has been shown to produce regression of adenomas in Familial Adenomatous Polyposis (FAP) patients (Muscat *et al*., 1994), although at least one study in sporadic adenomas has shown no such effect (Ladenheim *et al*., 1995).

The combination of DFMO and piroxicam has been shown to have a synergistic chemopreventive effect in the AOM-treated rat model of colon carcinogenesis (Reddy *et al*., 1990), although DFMO exerted a greater suppressive effect than piroxicam on Ki-ras mutation and tumorigenesis when each agent was administered separately (Reddy *et al*., 1990). In one study, administration of DFMO or piroxicam to AOM-treated rats reduced the number of tumors harboring Ki-ras mutations from 90% to 36% and 25% respectively (Singh *et al*., 1994). Both agents also reduced the amount of biochemically active p21 ras in existing tumors.

### C. Combinations of NSAIDs

Combinations of various NSAIDs are also used for various purposes. By using lower doses of two or more NSAIDs, it is possible to reduce the side effects or toxicities associated with higher doses of individual NSAIDs. For example, in some embodiments, sulindac may be used together with celecoxib. In some embodiments, the one or both of the NSAIDS are selective COX-2 inhibitors. Examples of NSAIDS that back be used either alone or in combination include, but are not limited to, the following: ibuprofen, naproxen, fenoprofen, ketoprofen, flurbiprofen, oxaprozin, indomethacin, sulindac, etodolac, diclofenac, piroxicam, meloxicam, tenoxicam, droxicam, lornoxicam, isoxicam, mefenamic acid, meclofenamic acid, flufenamic acid, tolfenamic acid, celecoxib rofecoxib valdecoxib parecoxib, lumiracoxib, or etoricoxib.

### IX. Eflornithine/Sulindac Combination Therapy

Preclinical studies of chemoprevention drugs given in combination at low doses show remarkable efficacy in preventing adenomas with little additional toxicities, suggesting a strategy to improve risk to benefit ratios for preventing recurrent adenomas.

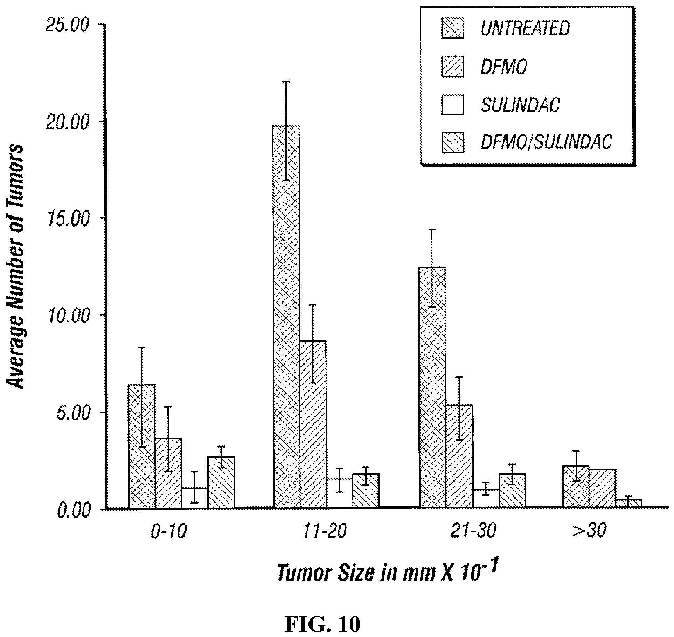

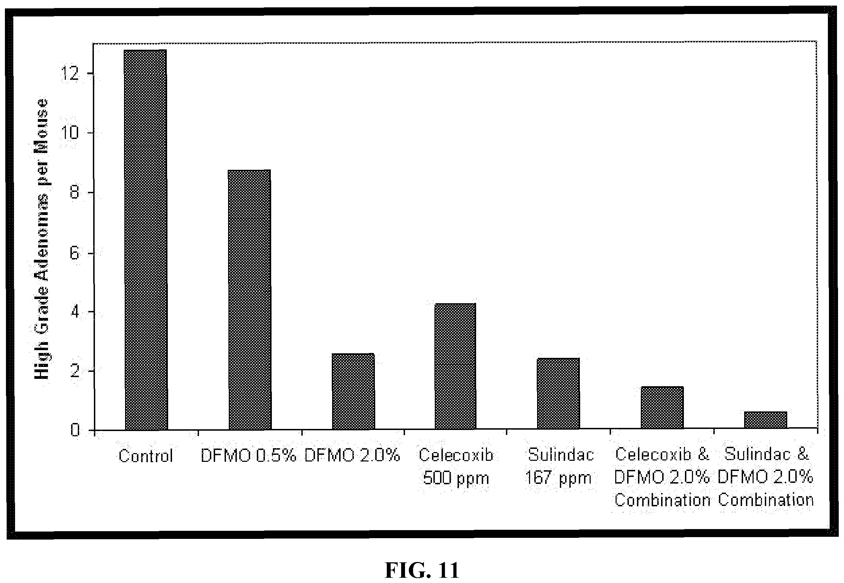

As noted above, the *Min* (multiple intestinal neoplasia) mouse, which shares a mutated *APC*/*apc* genotype with FAP, serves as a useful experimental animal model for human FAP patients (Lipkin, 1997). The *Min* mouse can develop greater than 100 gastrointestinal adenomas/adenocarcinomas throughout the gastrointestinal tract by 120 days of life leading to GI bleeding, obstruction and death. A combination therapy of DFMO and sulindac was shown to be effective in reducing adenomas in these mice (US Patent No. 6,258,845; Gerner and Meyskens, 2004). The results of treating *Min* mice with either DFMO alone, sulindac alone, or a combination of DFMO and sulindac on tumor formation in either the colon or small intestine are shown in FIGS. 9-10. FIG. 9 shows the average number of tumors by size in the colon of the three treatment groups compared to untreated controls. FIG. 10 shows the average number of tumors by size in the small intestine of the three treatment groups compared to untreated controls. FIG. 11 shows how the number of high grade adenomas various depending on therapy, single or combination.

### X. Efficacy of Polyamine-Inhibitory Therapy Based On Patient Profile

The efficacy of a polyamine-inhibitory combination of long-term daily oral *D,L*-α-difluoromethylornithine (DFMO, eflornithine) and sulindac among CRA patients was demonstrated (Meyskens *et al*., 2008), however, treatment was associated with modest, subclinical ototoxicity (McLaren *et al*., 2008), and a greater number of cardiovascular events among patients with high baseline cardiovascular risk (Zell *et al*., 2009). It has been determined that the *ODC1* genotype differentially affects adenoma recurrence, tissue polyamine responses, or toxicity profiles after eflornithine and sulindac treatment compared to placebo. U.S. Provisional Patent Application by Eugene Gerner, Jason Zell, Christine Mclaren, Frank Meyskens, Hoda Anton-Culver and Patricia A. Thompson, entitled "Carcinoma Diagnosis and Treatment, Based on ODC1 Genotype," filed May 14, 2010.

Three hundred seventy-five patients with history of resected (> or =3 mm) adenomas were randomly assigned to receive oral difluoromethylornithine (DFMO) 500 mg and sulindac 150 mg once daily or matched placebos for 36 months, stratified by use of low-dose aspirin (81 mg) at baseline and clinical site. Follow-up colonoscopy was done 3 years after randomization or off-study. Colorectal adenoma recurrence was compared among the groups with log-binomial regression. Comparing the outcome in patients receiving placebos to those receiving active intervention, (a) the recurrence of one or more adenomas was 41.1% and 12.3% (risk ratio, 0.30; 95% confidence interval, 0.18-0.49; P < 0.001); (b) 8.5% had one or more advanced adenomas, compared with 0.7% of patients (risk ratio, 0.085; 95% confidence interval, 0.011-0.65; P < 0.001); and (c) 17 (13.2%) patients had multiple adenomas (>1) at the final colonoscopy, compared with 1 (0.7%; risk ratio, 0.055; 0.0074-0.41; P < 0.001). Serious adverse events (grade > or =3) occurred in 8.2% of patients in the placebo group, compared with 11% in the active intervention group (P = 0.35). There was no significant difference in the proportion of patients reporting hearing changes from baseline. Recurrent adenomatous polyps can be markedly reduced by a combination of low oral doses of DFMO and sulindac and with few side effects. The details of this study are discussed in greater detail below and in Meyskens *et al*., 2008.

The study was halted by the Data Safety Monitoring Board (DSMB) after 267 patients completed end-of-study colonoscopies (due to the study meeting its efficacy endpoints). The DSMB monitored all safety and efficacy endpoints. As discussed in greater detail in the Examples, section this study involves analysis of patient data from the multicenter phase III colon adenoma prevention trial. See also (Meyskens *et al*., 2008).

### A. ODC1 Genotype Distribution

A total of 440 colorectal cancer (CRC) cases identified from the UC Irvine CRC gene-environment study were used in the case-only analysis. Median follow-up duration was 11 years. There were 270 (61%) colon cancer cases, 162 (37%) rectal cancer cases, and 8 (2%) CRC cases of unspecified location. Clinicopathologic data for colon and rectal cancer cases are shown in Table 1. ODC +316 genotype distribution among all CRC cases was 53% GG, 41% GA, and 7% AA. ODC +316 genotype distribution was similar among CRC cases with and without a family history. There were no significant differences in ODC genotype distribution by age (P = 0.38), gender (P = 0.56), family history (P = 0.94), site within the colorectum (P = 0.55), histology (P = 0.46) or tumor grade (P = 0.73). ODC genotype distribution did not significantly differ by stage at diagnosis: stage I (49% GG, 42% GA, 8% AA), stage II (56% GG, 38% GA, 6% AA), stage III (51% GG, 43% GA, 6% AA), stage IV (59% GG, 37% GA, 4% AA) (P = 0.87). ODC genotype distribution by ethnicity revealed significant differences: Caucasian (382 cases: 53% GG, 41% GA, 6% AA, minor-A allele frequency = 26%), African-American (7 cases: 71% GG, 29% GA, 0% AA, minor-A allele frequency = 15%), Hispanics (21 cases: 57% GG, 43% GA, 0% AA, minor-A allele frequency = 21%), and Asians (27 cases: 33% GG, 41% GA, 26% AA, minor-A allele frequency = 46%) (P = 0.009). However, within each race ODC genotype distribution was in Hardy-Weinberg equilibrium (Caucasians P = 0.36, African-Americans P = 0.66, Hispanics P = 0.21, Asians P = 0.35).

### B. Adenoma Recurrence

*ODC1* genotype distribution was: 126 GG (55%), 87 GA (38%), and 15 AA (7%). Baseline clinical characteristics revealed differences, as shown in Table 1. In regression models with predictors age, gender, race, aspirin use, treatment, ODC1 genotype, and treatment, treatment was the only factor associated with differences in adenoma recurrence, tissue polyamine response, and ototoxicity. A statistically significant interaction was detected for ODC1 genotype and treatment in the full model for adenoma recurrence (P = 0.021), such that the pattern of adenoma recurrence among placebo patients was: GG-50%, GA-35%, AA-29% versus eflornithine/sulindac patients: GG-11%, GA-14%, AA-57%.

A statistically significant interaction was detected between *ODC1* genotype and treatment in this model (P = 0.038). *ODC1* genotype was not significantly associated with a tissue putrescine response or spermidine:spermine ratio response in the full regression models (data not shown). The relative risk (RR) for adenoma recurrence related to treatment after adjustment in the full regression model was 0.39 (95% CI 0.24-0.66). There were no significant associations between treatment and *ODC1* genotype group with regard to cardiovascular or gastrointestinal adverse events (Tables 3 & 4).

Here it was observed that the adenoma-inhibitory effect of eflornithine and sulindac was greater among those with the major G homozygous *ODC1* genotype, in contrast to prior reports showing decreased risk of recurrent adenoma among CRA patients receiving aspirin carrying at least one A-allele (Martinez *et al*., 2003; Barry *et al*., 2006; Hubner *et al*., 2008) *ODC1* genotype distribution was similar to that reported in prior aspirin-based trials (Martinez *et al*., 2003; Barry *et al*., 2006; Hubner *et al*., 2008), and the A-allele was associated with a non-significant lower recurrent adenoma risk in the placebo group consistent with previous reports (Martinez *et al*., 2003; Hubner *et al*., 2008). These results demonstrate that *ODC1* A-allele carriers differ in response to prolonged exposure with eflornithine and sulindac compared to GG genotype patients, with A-allele carriers experiencing less benefit in terms of adenoma recurrence, and potential for elevated risk of developing ototoxicity, especially among the AA homozygotes.

### C. Survival Analysis

Of the 440 CRC cases, 138 (31%) were deceased at the time of analysis. Sixty-four (46%) deaths occurred in cases carrying the GG genotype, compared to 74 (54%) deaths in cases with the AA/AG genotypes. Cause of death was available for 102 of the 138 deceased CRC cases. Eighty-five (83%) CRC cases died as a result of CRC. A statistically significant improvement in CRC-specific survival was observed among all CRC cases homozygous for the ODC G-allele (10-year survival = 84%) compared to cases with at least one A-allele (ODC GA/AA) (10-year survival = 76%; P = 0.031). CRC-specific survival analysis by stage revealed that significantly different survival differences were not observed for AJCC stage I (P = 0.055), II (P = 0.61), or IV (P = 0.65) CRC. However, among cases with stage III CRC the ODC GG genotype was associated with improved 10-year CRC-specific survival: 75% compared to 60% for ODC GA/AA genotype cases; P = 0.024 (FIG. 1). Among colon cancer cases, a statistically significant CRC-specific survival benefit was observed for those with ODC GG genotype compared to ODC GA/AA cases (10-year survival rate = 87% vs. 79%; P = 0.029); this was not observed for rectal cancer cases (10-year survival = 78% for ODC GG cases vs. 72% for ODC GA/AA cases; P = 0.42).

Among all CRC cases, the CRC-specific survival estimates based on ODC genotype after adjustment for age (years), gender, ethnicity, family history of CRC, TNM stage at diagnosis, tumor site within the colon, histologic subtype, treatment with surgery, radiation therapy, and chemotherapy were a follows: ODC GG hazards ratio (HR) = 1.00 (referent), ODC GA HR = 1.73, and ODC AA genotype HR = 1.73 (P-trend = 0.0283). Among colon cases only, CRC-specific survival analysis revealed that the ODC +316 SNP was an independent predictor of CRC-specific survival, after adjustment for the above clinical variables. Compared to ODC GG colon cancer cases, the CRC-specific risk of death (HR) was 2.31 (1.15-4.64) for ODC GA genotype and 3.73 (0.93-14.99) for ODC AA genotype (P-trend = 0.006) (Table 2). Overall survival analysis of these colon cancer cases was consistent with the CRC-specific survival analysis (Table 2). Among rectal cancer cases, CRC-specific survival analysis revealed that the ODC +316 SNP was not an independent predictor of CRC-specific survival after adjustment for the aforementioned clinical variables. Compared to ODC GG rectal cancer cases (HR=1.00, reference), the CRC-specific risk of death (HR) was 1.72 (0.83-3.57) for ODC GA heterozygotes and 1.93 (0.56-6.67) for ODC AA homozygotes (P-trend = 0.12).

As noted above, the ODC +316 genotype distribution differed across ethnicity. The observed mortality risk, other than by chance, likely reflects differences based on ODC genotype, however the risk may be restricted to a particular ethnic group. Thus multivariate analyses were conducted among Caucasian colon cancer cases, to assess genotype-specific mortality risk within this single ethnic group. Among the 234 Caucasian colon cancer cases, there were 37 CRC-related deaths. Multivariate CRC-specific survival analysis revealed that the ODC +316 SNP was an independent predictor of CRC-specific survival among Caucasian colon cancer cases after adjustment for the aforementioned relevant clinical variables. Compared to cases with ODC GG genotype (HR=1.00, reference), the CRC-specific risk of death (HR) was 2.67 (1.22-5.82) for ODC GA genotype and 6.28 (1.46-26.95) for ODC AA genotype (P-trend = 0.0018).

Genotype-specific survival differences among CRC cases were limited to colon cancer cases: compared to ODC GG genotype cases (HR=1.00, reference) the adjusted CRC-SS hazards ratio (HR) was 2.31 (1.15-4.64) for ODC GA cases and 3.73 (0.93-14.99) for ODC AA cases (P-trend=0.006). In colon cancer cells, the ODC +316 SNP, flanked by two E-boxes, predicts ODC promoter activity. The E-box activator *c-MYC* and repressors *MAD1* and MAD4 preferentially bind to minor A-, compared to major G-, alleles in cultured cells.

Based on this population-based analysis of colorectal cancer cases with eleven years follow-up duration, it was observed that the +316 ODC SNP was associated with colorectal cancer specific survival among colon cancer cases. A statistically significant increased risk of CRC-specific mortality was observed with each additional ODC A-allele among colon cancer cases, *i.e.,* from ODC GG to GA to AA (P-trend=0.006), after adjustment for age, gender, ethnicity, tumor stage, family history of CRC, tumor site, histology, treatment with surgery, radiation therapy, and chemotherapy.

### D. Allele Specific Regulation of Transcription Factors

In colon cancer epithelial cells, it has been shown that the ODC +316 SNP is functionally significant, as evidenced by increased binding of E-box transcription factors to promoter elements containing A-, compared to G-, alleles. Both the activator *c-MYC* and the repressor *MAD1* show greater effects on promoter activity in reporter elements containing A-versus G- alleles. These results suggest allele-specific regulation of ODC by E-box transcription factors. ODC protein enzyme activity is not apparently affected by the ODC +316 SNP genotype, which we believe influences ODC transcription.

In colon cells, it has been shown that conditional expression of wild type *APC,* a gene expressed in normal colonic mucosa, suppresses *c-MYC,* and increases *MAD1,* expression (Fultz and Gerner, 2002). Further, it has have reported that wild type *APC* can regulate ODC promoter activity in a manner dependent on the +316 SNP (Martinez *et al*., 2003). Wild type *APC* is expressed in the apparently normal colonic mucosa of individuals not afflicted with FAP, while the majority of sporadic colon adenomas show evidence of mutated or deleted *APC* (Iwamoto *et al*., 2000). MYC is expressed at low levels in normal intestinal mucosa but is increased in intestinal adenomas of *APC*^{*Min*/+} mice. Conditional knockout of intestinal epithelial MYC expression suppresses intestinal tumorigenesis in *APC*^{*Min*/+} mice (Ignatenko *et al*., 2006). As described above, previous work by our group (Martinez *et al*., 2003) and others (Hubner *et al*., 2008) demonstrated a protective role for the ODC A-allele, especially in aspirin users, against recurrence of colon polyps in clinical prevention trials. However, in the population-based study presented here, the ODC A-allele was associated with poor survival. This apparent contradiction may be explained by the results shown here, which indicate that both E-box activators and repressors bind the ODC A-allele selectively. The transition from normal epithelium, expressing E-box repressors, to neoplastic epithelium may be retarded in individuals with ODC A-alleles. This effect may result from suppression of polyamine synthesis. However, if the transformed epithelium begins to express E-box activators (such as *c-MYC*), then cancer progression may be more likely to occur in individuals with the ODC A genotype. The results for risk of colon cancer-specific mortality are consistent with those showing that risk of prostate cancer may be associated with the ODC A-allele among specific individuals as the result of gene environment interactions (O'Brien *et al*., 2004; Visvanathan *et al*., 2004). Such colon cancer progression could be due to enhanced polyamine synthesis, as has been demonstrated already for prostate cancer (Simoneau *et al*., 2008).

This finding that a factor, such as the ODC SNP, may have both promoting and inhibiting effects on carcinogenesis is not unique. For example, transforming growth factor-beta (TGF-β) has diverse roles in carcinogenesis and cancer progression (Derynck *et al*., 2001; Pardali and Moustakas, 2007; Roberts and Wakefield, 2003). TGF-β in untransformed cells inhibits cell proliferation and induces apoptosis. Yet, it is overexpressed in all human tumors and is associated with late cancer progression, specifically tumor invasion and metastasis. A single study reporting ODC activity in human colorectal tumors demonstrated that high levels of ODC expression was significantly associated with improved survival (Matsubara *et al*., 1995). This suggests that, although ODC overexpression promotes the formation of human colorectal adenomas, it is possible that in established lesions, ODC overexpression causes enhanced proliferation and is associated with improved response to anti-proliferative treatments. However, that study did not include stratification by ODC genotype, so it is not known if these effects are independent of ODC genotype.

The observed associations of the ODC +316 SNP with CRC-specific mortality were limited to colon cancer cases. Among colon cancer cases, particularly strong effects were observed for Caucasians. Similar to other reports, the ODC +316 SNP allele frequency differs considerably by ethnicity (O'Brien *et al*., 2004). When the survival analysis was limited to Caucasians only (*i.e.,* the only ethnic group with adequate power for such analyses), the associations of the ODC +316 SNP were significant, and of greater magnitude than the estimates observed for the entire cohort.

The epidemiologic study shares limitations of other population-based analyses, including lack of data on comorbid conditions, performance status, and particular chemotherapeutic regimens utilized. Additionally, the tissue biopsy samples obtained from participants of the UC Irvine Gene-Environment Study of Familial Colorectal Cancer are paraffin-embedded specimens and therefore cannot be used for accurate assessment of tissue polyamine quantification by high performance liquid chromatography (HPLC). There is also the potential for selection bias, favoring a relatively healthy group of CRC survivors, since there was a median 16 month delay from the time of CRC diagnosis until study enrollment. Other factors affecting polyamine metabolism that were not accounted for in the present study may explain our observations. For example, aspirin activates polyamine acetylation and export and works with the ODC A-allele to reduce cell and tissue polyamine contents (Gerner *et al*., 2004; Martinez *et al*., 2003; Babbar *et al*., 2006).

In summary, clinical consequences of the ODC +316 SNP on CRC-specific mortality among colon cancer cases have been observed. Additionally, the functional significance of the ODC +316 SNP in the *c-MYC-* and *MAD1*-dependent transcription of this gene in human colon cancer cells is better understood. Together, these experimental and epidemiologic findings suggest roles for the ODC +316 SNP in progression of colon cancer that are distinct from its previously reported role in progression to colon adenomas. These findings may be used to assess risk of colon cancer progression and may be used to direct patient-specific pharmacogenetic management, surveillance monitoring, and inform novel targeted approaches to secondary and tertiary colon cancer prevention.

### E. Summary

A statistically significant interaction was detected for ODC1 genotype and treatment in the full model for adenoma recurrence (*P* = 0.021), such that the pattern of adenoma recurrence among placebo patients was: GG-50%, GA-35%, AA-29% versus eflornithine/sulindac patients: GG-11%, GA-14%, AA-57%. Here it was observed that the adenoma-inhibitory effect of eflornithine and sulindac was greater among those with the major G homozygous *ODC1* genotype, in contrast to prior reports showing decreased risk of recurrent adenoma among CRA patients receiving aspirin carrying at least one A-allele (Martinez *et al*., 2003; Barry *et al*., 2006; Hubner *et al*., 2008) These results demonstrate that *ODC1* A-allele carriers differ in response to prolonged exposure with eflornithine and sulindac compared to GG genotype patients, with A-allele carriers experiencing less benefit in terms of adenoma recurrence, and potential for elevated risk of developing ototoxicity, especially among the AA homozygotes.

### XI. Interaction between Treatment with DFMO and Sulindac and Dietary Polyamine Intake

In a study described in greater detail in the Examples section below, the inventors observed a significant interaction between treatment with DFMO + sulindac and dietary polyamine intake on the risk of recurrent adenomas in a colorectal adenoma prevention trial. Patients in the highest quartile of dietary polyamine intake exhibited no metachronous adenoma risk reduction after treatment with DFMO + sulindac, in contrast to an 81% risk reduction observed for patients in the lower three quartiles of polyamine intake.

In this study, patients in the highest dietary polyamine intake group had higher tissue polyamine levels, and a greater proportion of large adenomas (43.6% *versus* 26.4% *P* = 0.016) and advanced adenomas (52.7% *versus* 35.9% *P* = 0.028) at baseline (Table 2). Additionally, a greater proportion of patients in the highest dietary polyamine intake group had high grade adenomas (32.7% *versus* 20.4% *P* = 0.060), although this difference was not significant. These results generally reflect the results of mouse model studies where increased dietary polyamine intake has been significantly associated with increasing grade of intestinal adenoma dysplasia. Ignatenko NA, Besselsen DG, Roy UK, et al.: Dietary putrescine reduces the intestinal anticarcinogenic activity of sulindac in a murine model of familial adenomatous polyposis. Nutr Cancer 56:172-81, 2006. In another animal study, it was observed that decreasing the polyamine absorption by dietary administration of *Bifidobacterium longum* resulted in significant suppression of colon tumor incidence, tumor multiplicity, and reduction of tumor size by inhibiting the cell proliferation rate as well as ODC activity. Singh J, Rivenson A, Tomita M, et al.: Bifidobacterium longum, a lactic acid-producing intestinal bacterium inhibits colon cancer and modulates the intermediate biomarkers of colon carcinogenesis. Carcinogenesis 18:833-41, 1997.

These results demonstrate elevated baseline tissue spermine and spermidine levels in patients within the highest quartile of dietary polyamine intake. These findings generally concur with experimental studies done by Ignatenko, in which dietary putrescine supplementation increased intestinal tissue putrescine levels in APC ^{Min/+} mice. Ignatenko NA, Besselsen DG, Roy UK, et al.: Dietary putrescine reduces the intestinal anticarcinogenic activity of sulindac in a murine model of familial adenomatous polyposis. Nutr Cancer 56:172-81, 2006. However, tissue putrescine levels were not associated with baseline dietary polyamine intake in the present analysis. This could be because only 10% of dietary putrescine is retained in body tissues as opposed to 40% of dietary spermidine. Hughes EL, Grant G, Pusztai A, et al.: Uptake and inter-organ distribution of dietary polyamines in the rat. Biochem Soc Trans 26:S369, 1998. Externally-obtained polyamines are transported into cells from the extracellular spaces and once inside the cell, exogenous putrescine is rapidly converted into spermidine and spermine (which are interconvertible). Milovic V, Turhanowa L, Fares FA, et al.: S-adenosylmethionine decarboxylase activity and utilization of exogenous putrescine are enhanced in colon cancer cells stimulated to grow by EGF. Z Gastroenterol 36:947-54, 1998.

One of the aims of the study was to evaluate the interaction of dietary polyamine intake and treatment with DFMO + sulindac on metachronous adenoma risk reduction, and a statistically significant interaction was observed (P = 0.01). Here, the inventors report an 81% risk reduction of metachronous adenomas in the lower dietary polyamine group by DFMO + sulindac, a substantial risk reduction compared to the parent study in which a 70% adenoma risk reduction was observed. This underscores the potential utility of restricting dietary polyamine intake in prevention of colorectal neoplasia. They also noted a 94% risk reduction of advanced adenomas in the lower dietary polyamine group, which was similar to that reported in the parent study. In the highest dietary polyamine group, however, no benefit with DFMO + sulindac (*versus* placebo) was observed [RR, 1.04; 95% CI, 0.32-3.36]. In murine experimental studies, inhibition of ODC, induction of spermidine/spermine N1-acetyltransferase, and induction of polyamine export by NSAIDs contributed to decreased intracellular polyamine content and apoptosis. Addition of spermidine to the cells prevents apoptosis and restores cell number, suggesting that exogenous polyamines could reverse the efficacy of NSAIDs as observed in our study. Ignatenko NA, Besselsen DG, Roy UK, et al.: Dietary putrescine reduces the intestinal anticarcinogenic activity of sulindac in a murine model of familial adenomatous polyposis. Nutr Cancer 56:172-81, 2006; Hughes A, Smith NI, Wallace HM: Polyamines reverse non-steroidal anti-inflammatory drug-induced toxicity in human colorectal cancer cells. Biochem J 374:481-8, 2003. These data reinforce results from murine experiments showing that exogenous polyamines govern polyamine homeostasis as well as influence the efficacy of DFMO and sulindac. The reduction in risk of adenoma recurrence from DFMO + sulindac *versus* placebo in the lower dietary polyamine group (*i.e*., 3/4^{ths} of the study population) confirms ODC inhibition as a promising approach in colon cancer chemoprevention in this group.

Other sources of polyamine regulation should be considered. Genetic polymorphism in *ODC1* has been shown to be associated with colorectal cancer survival, and may influence the adenoma recurrence risk after treatment with DFMO + sulindac. Zell JA, Ziogas A, Ignatenko N, et al.: Associations of a polymorphism in the ornithine decarboxylase gene with colorectal cancer survival. Clin Cancer Res 15:6208-16, 2009; Zell JA, McLaren CE, Chen W-P, et al.: Ornithine decarboxylase (Odc)-1 gene polymorphism effects on baseline tissue polyamine levels and adenoma recurrence in a randomized phase III adenoma prevention trial of DFMO + sulindac versus placebo. J Clin Oncol 26S:Abstract 1502, 2008. Interaction between *ODC1* genotype and dietary polyamine intake may therefore affect polyamine homeostasis. However, the inventors do not have adequate power to stratify the present analysis by ODC genotype.

Here, the inventors validate the previously-established polyamine database by demonstrating the relationship between dietary polyamine intake and tissue polyamine levels at baseline. Polyamine content is high in several food products, including nuts, certain cheeses, and meat. Although meat consumption may be a surrogate for polyamine consumption, substantial amounts of dietary polyamines are also obtained from various foods such as orange juice, corn, and peas. The observed association for total polyamine intake and total daily arginine (*r*ₛ = 0.64) was greater than that observed for animal-derived protein intake (*r*ₛ = 0.49). This relationship between arginine and dietary polyamines supports prior research in *APC*^{*Min*/+} mice showing that dietary arginine-induced intestinal tumorigenesis can be inhibited by polyamine-inhibitory agents such as DFMO and NSAIDs. Zell JA, Ignatenko NA, Yerushalmi HF, et al.: Risk and risk reduction involving arginine intake and meat consumption in colorectal tumorigenesis and survival. Int J Cancer 120:459-68, 2007.

There are some limitations to this study. The main limitation is the relatively small sample size (n = 188) in the analysis of recurrence risk. With such small numbers, this study did not have sufficient power to perform detailed subset analyses, especially in the highest dietary polyamine group. Also, it must be acknowledged that the study design did not include stratification based on baseline dietary polyamine intake. It is acknowledged that the dietary habits of people can change with time, which could influence our results- especially given that our analysis is based on the baseline dietary questionnaire and not at various time intervals during the study. However, it is worth noting that there is presumed to be a low likelihood of major dietary changes among middle aged to older adults during the 3 years on-study in the absence of a life-threatening new medical condition, as patients in this age group typically have a stable and consistent eating pattern. Therefore, using data from the single initial FFQ in the analysis has validity. All dietary assessment methodologies based on self-report have limitations in accuracy and at best can separate high intake from low intake (as analyzed in this study), rather than providing precise intake measurements. Almost 50% of patients were taking aspirin, which could modulate the polyamine body pool; however, the final estimates were adjusted for aspirin use and we did not observe a significant interaction between baseline dietary polyamine intake and aspirin in the logistic regression model.

In the parent study, a prospective, randomized, placebo-controlled clinical trial of combination of DFMO (a selective inhibitor of polyamine synthesis) and sulindac (a NSAID) over a 3-year treatment duration resulted in a 70% reduction in recurrence of all metachronous adenomas and >90% reduction in recurrence of advanced and multiple adenomas compared to placebo. The findings presented in this dietary polyamine analysis, demonstrating a 81% risk reduction in metachronous adenomas and 94% reduction in advanced adenomas among patients reporting low-normal dietary polyamine intake suggest that dietary polyamines may be an important factor in adenoma prevention. Controlling exogenous polyamines may be used as an adjunctive strategy to chemoprevention with polyamine inhibitory agents, for example, as secondary and tertiary colorectal cancer prevention trials utilizing polyamine-inhibitory agents are developed.

### XII. Polymorphism Analysis

The genotype at the +316 position of the *ODC1* promoter gene of patient can determined using the methods provided below, including the specific methods described in the Examples section. These methods can be further modified and optimized using the principles and techniques of molecular biology as applied by a person skilled in the art. Such principles and techniques are taught, for example, in Small *et al*., (2002). General methods employed for the identification of single nucleotide polymorphisms (SNPs) are provided below. The reference of Kwok and Chen (2003) and Kwok (2001) provide overviews of some of these methods.

SNPs relating to *ODC1* can be characterized by the use of any of these methods or suitable modification thereof. Such methods include the direct or indirect sequencing of the site, the use of restriction enzymes where the respective alleles of the site create or destroy a restriction site, the use of allele-specific hybridization probes, the use of antibodies that are specific for the proteins encoded by the different alleles of the polymorphism, or any other biochemical interpretation.

### A. DNA Sequencing

A commonly used method of characterizing a polymorphism is direct DNA sequencing of the genetic locus that flanks and includes the polymorphism. Such analysis can be accomplished using either the "dideoxy-mediated chain termination method," also known as the "Sanger Method" (Sanger *et al*., 1975) or the "chemical degradation method," also known as the "Maxam-Gilbert method" (Maxam *et al*., 1977). Sequencing in combination with genomic sequence-specific amplification technologies, such as the polymerase chain reaction may be utilized to facilitate the recovery of the desired genes (Mullis *et al*., 1986; European Patent Application 50,424; European Patent Application. 84,796, European Patent Application 258,017, European Patent Application. 237,362; European Patent Application. 201,184; U.S. Patents 4,683,202; 4,582,788; and 4,683,194).

### B. Exonuclease Resistance

Other methods that can be employed to determine the identity of a nucleotide present at a polymorphic site utilize a specialized exonuclease-resistant nucleotide derivative (U.S. Patent. 4,656,127). A primer complementary to an allelic sequence immediately 3'-to the polymorphic site is hybridized to the DNA under investigation. If the polymorphic site on the DNA contains a nucleotide that is complementary to the particular exonucleotide-resistant nucleotide derivative present, then that derivative will be incorporated by a polymerase onto the end of the hybridized primer. Such incorporation makes the primer resistant to exonuclease cleavage and thereby permits its detection. As the identity of the exonucleotide-resistant derivative is known one can determine the specific nucleotide present in the polymorphic site of the DNA.

### C. Microsequencing Methods

Several other primer-guided nucleotide incorporation procedures for assaying polymorphic sites in DNA have been described (Komher *et al*., 1989; Sokolov, 1990; Syvanen 1990; Kuppuswamy *et al*., 1991; Prezant *et al*., 1992; Ugozzoll *et al*., 1992; Nyren *et al*., 1993). These methods rely on the incorporation of labeled deoxynucleotides to discriminate between bases at a polymorphic site. As the signal is proportional to the number of deoxynucleotides incorporated, polymorphisms that occur in runs of the same nucleotide result in a signal that is proportional to the length of the run (Syvanen *et al*.,1990).

### D. Extension in Solution

French Patent 2,650,840 and PCT Application WO91/02087 discuss a solution-based method for determining the identity of the nucleotide of a polymorphic site. According to these methods, a primer complementary to allelic sequences immediately 3'-to a polymorphic site is used. The identity of the nucleotide of that site is determined using labeled dideoxynucleotide derivatives which are incorporated at the end of the primer if complementary to the nucleotide of the polymorphic site.

### E. Genetic Bit Analysis or Solid-Phase Extension

PCT Application WO92/15712 describes a method that uses mixtures of labeled terminators and a primer that is complementary to the sequence 3' to a polymorphic site. The labeled terminator that is incorporated is complementary to the nucleotide present in the polymorphic site of the target molecule being evaluated and is thus identified. Here the primer or the target molecule is immobilized to a solid phase.

### F. Oligonucleotide Ligation Assay (OLA)

This is another solid phase method that uses different methodology (Landegren *et al*., 1988). Two oligonucleotides, capable of hybridizing to abutting sequences of a single strand of a target DNA are used. One of these oligonucleotides is biotinylated while the other is detectably labeled. If the precise complementary sequence is found in a target molecule, the oligonucleotides will hybridize such that their termini abut, and create a ligation substrate. Ligation permits the recovery of the labeled oligonucleotide by using avidin. Other nucleic acid detection assays, based on this method, combined with PCR have also been described (Nickerson *et al*., 1990). Here PCR is used to achieve the exponential amplification of target DNA, which is then detected using the OLA.

### G. Ligase/Polymerase-Mediated Genetic Bit Analysis

U.S. Patent 5,952,174 describes a method that also involves two primers capable of hybridizing to abutting sequences of a target molecule. The hybridized product is formed on a solid support to which the target is immobilized. Here the hybridization occurs such that the primers are separated from one another by a space of a single nucleotide. Incubating this hybridized product in the presence of a polymerase, a ligase, and a nucleoside triphosphate mixture containing at least one deoxynucleoside triphosphate allows the ligation of any pair of abutting hybridized oligonucleotides. Addition of a ligase results in two events required to generate a signal, extension and ligation. This provides a higher specificity and lower "noise" than methods using either extension or ligation alone and unlike the polymerase-based assays, this method enhances the specificity of the polymerase step by combining it with a second hybridization and a ligation step for a signal to be attached to the solid phase.

### H. Invasive Cleavage Reactions

Invasive cleavage reactions can be used to evaluate cellular DNA for a particular polymorphism. A technology called INVADER® employs such reactions (*e.g.,* de Arruda *et al*., 2002; Stevens *et al*., 2003). Generally, there are three nucleic acid molecules: 1) an oligonucleotide upstream of the target site ("upstream oligo"), 2) a probe oligonucleotide covering the target site ("probe"), and 3) a single-stranded DNA with the the target site ("target"). The upstream oligo and probe do not overlap but they contain contiguous sequences. The probe contains a donor fluorophore, such as fluoroscein, and an acceptor dye, such as Dabcyl. The nucleotide at the 3' terminal end of the upstream oligo overlaps ("invades") the first base pair of a probe-target duplex. Then the probe is cleaved by a structure-specific 5' nuclease causing separation of the fluorophore/quencher pair, which increases the amount of fluorescence that can be detected. *See* Lu *et al*., 2004.

In some cases, the assay is conducted on a solid-surface or in an array format.

### 1. Other Methods To Detect SNPs

Several other specific methods for polymorphism detection and identification are presented below and may be used as such or with suitable modifications in conjunction with identifying polymorphisms of the *ODC1* gene in the present invention. Several other methods are also described on the SNP web site of the NCBI on the World Wide Web at ncbi.nlm.nih.gov/SNP.

In a particular aspect, extended haplotypes may be determined at any given locus in a population, which allows one to identify exactly which SNPs will be redundant and which will be essential in association studies. The latter is referred to as 'haplotype tag SNPs (htSNPs)', markers that capture the haplotypes of a gene or a region of linkage disequilibrium. See Johnson *et al.* (2001) and Ke and Cardon (2003), for exemplary methods.

The VDA-assay utilizes PCR amplification of genomic segments by long PCR methods using TaKaRa LA Taq reagents and other standard reaction conditions. The long amplification can amplify DNA sizes of about 2,000-12,000 bp. Hybridization of products to variant detector array (VDA) can be performed by a Affymetrix High Throughput Screening Center and analyzed with computerized software.

A method called Chip Assay uses PCR amplification of genomic segments by standard or long PCR protocols. Hybridization products are analyzed by VDA, Halushka et al. (1999). SNPs are generally classified as "Certain" or "Likely" based on computer analysis of hybridization patterns. By comparison to alternative detection methods such as nucleotide sequencing, "Certain" SNPs have been confirmed 100% of the time; and "Likely" SNPs have been confirmed 73% of the time by this method.

Other methods simply involve PCR amplification following digestion with the relevant restriction enzyme. Yet others involve sequencing of purified PCR products from known genomic regions.

In yet another method, individual exons or overlapping fragments of large exons are PCR-amplified. Primers are designed from published or database sequences and PCR-amplification of genomic DNA is performed using the following conditions: 200 ng DNA template, 0.5 µM each primer, 80µM each of dCTP, dATP, dTTP and dGTP, 5% formamide, 1.5mM MgCl₂, 0.5 U of Taq polymerase and 0.1 volume of the Taq buffer. Thermal cycling is performed and resulting PCR-products are analyzed by PCR-single strand conformation polymorphism (PCR-SSCP) analysis, under a variety of conditions, e.g, 5 or 10% polyacrylamide gel with 15% urea, with or without 5% glycerol. Electrophoresis is performed overnight. PCR-products that show mobility shifts are reamplified and sequenced to identify nucleotide variation.

In a method called CGAP-GAI (DEMIGLACE), sequence and alignment data (from a PHRAP.ace file), quality scores for the sequence base calls (from PHRED quality files), distance information (from PHYLIP dnadist and neighbour programs) and base-calling data (from PHRED '-d' switch) are loaded into memory. Sequences are aligned and examined for each vertical chunk ('slice') of the resulting assembly for disagreement. Any such slice is considered a candidate SNP (DEMIGLACE). A number of filters are used by DEMIGLACE to eliminate slices that are not likely to represent true polymorphisms. These include filters that: (i) exclude sequences in any given slice from SNP consideration where neighboring sequence quality scores drop 40% or more; (ii) exclude calls in which peak amplitude is below the fifteenth percentile of all base calls for that nucleotide type; (iii) disqualify regions of a sequence having a high number of disagreements with the consensus from participating in SNP calculations; (iv) removed from consideration any base call with an alternative call in which the peak takes up 25% or more of the area of the called peak; (v) exclude variations that occur in only one read direction. PHRED quality scores were converted into probability-of-error values for each nucleotide in the slice. Standard Baysian methods are used to calculate the posterior probability that there is evidence of nucleotide heterogeneity at a given location.

In a method called CU-RDF (RESEQ), PCR amplification is performed from DNA isolated from blood using specific primers for each SNP, and after typical cleanup protocols to remove unused primers and free nucleotides, direct sequencing using the same or nested primers.

In a method called DEBNICK (METHOD-B), a comparative analysis of clustered EST sequences is performed and confirmed by fluorescent-based DNA sequencing. In a related method, called DEBNICK (METHOD-C), comparative analysis of clustered EST sequences with phred quality > 20 at the site of the mismatch, average phred quality >= 20 over 5 bases 5'-FLANK and 3' to the SNP, no mismatches in 5 bases 5' and 3' to the SNP, at least two occurrences of each allele is performed and confirmed by examining traces.

In a method identified by ERO (RESEQ), new primers sets are designed for electronically published STSs and used to amplify DNA from 10 different mouse strains. The amplification product from each strain is then gel purified and sequenced using a standard dideoxy, cycle sequencing technique with ³³P-labeled terminators. All the ddATP terminated reactions are then loaded in adjacent lanes of a sequencing gel followed by all of the ddGTP reactions and so on. SNPs are identified by visually scanning the radiographs.

In another method identified as ERO (RESEQ-HT), new primers sets are designed for electronically published murine DNA sequences and used to amplify DNA from 10 different mouse strains. The amplification product from each strain is prepared for sequencing by treating with Exonuclease I and Shrimp Alkaline Phosphatase. Sequencing is performed using ABI Prism Big Dye Terminator Ready Reaction Kit (Perkin-Elmer) and sequence samples are run on the 3700 DNA Analyzer (96 Capillary Sequencer).

FGU-CBT (SCA2-SNP) identifies a method where the region containing the SNP were PCR amplified using the primers SCA2-FP3 and SCA2-RP3. Approximately 100 ng of genomic DNA is amplified in a 50 ml reaction volume containing a final concentration of 5 mM Tris, 25 mM KCl, 0.75 mM MgCl₂, 0.05% gelatin, 20 pmol of each primer and 0.5U of Taq DNA polymerase. Samples are denatured, annealed and extended and the PCR product is purified from a band cut out of the agarose gel using, for example, the QIAquick gel extraction kit (Qiagen) and is sequenced using dye terminator chemistry on an ABI Prism 377 automated DNA sequencer with the PCR primers.

In a method identified as JBLACK (SEQ/RESTRICT), two independent PCR reactions are performed with genomic DNA. Products from the first reaction are analyzed by sequencing, indicating a unique FspI restriction site. The mutation is confirmed in the product of the second PCR reaction by digesting with Fsp I.

In a method described as KWOK(1), SNPs are identified by comparing high quality genomic sequence data from four randomly chosen individuals by direct DNA sequencing of PCR products with dye-terminator chemistry (see Kwok *et al*., 1996). In a related method identified as KWOK(2) SNPs are identified by comparing high quality genomic sequence data from overlapping large-insert clones such as bacterial artificial chromosomes (BACs) or PI-based artificial chromosomes (PACs). An STS containing this SNP is then developed and the existence of the SNP in various populations is confirmed by pooled DNA sequencing (see Taillon-Miller *et al.,* 1998). In another similar method called KWOK(3), SNPs are identified by comparing high quality genomic sequence data from overlapping large-insert clones BACs or PACs. The SNPs found by this approach represent DNA sequence variations between the two donor chromosomes but the allele frequencies in the general population have not yet been determined. In method KWOK(5), SNPs are identified by comparing high quality genomic sequence data from a homozygous DNA sample and one or more pooled DNA samples by direct DNA sequencing of PCR products with dye-terminator chemistry. The STSs used are developed from sequence data found in publicly available databases. Specifically, these STSs are amplified by PCR against a complete hydatidiform mole (CHM) that has been shown to be homozygous at all loci and a pool of DNA samples from 80 CEPH parents (see Kwok *et al.,* 1994).

In another such method, KWOK (OverlapSnpDetectionWithPolyBayes), SNPs are discovered by automated computer analysis of overlapping regions of large-insert human genomic clone sequences. For data acquisition, clone sequences are obtained directly from large-scale sequencing centers. This is necessary because base quality sequences are not present/available through GenBank. Raw data processing involves analyzed of clone sequences and accompanying base quality information for consistency. Finished ('base perfect', error rate lower than 1 in 10,000 bp) sequences with no associated base quality sequences are assigned a uniform base quality value of 40 (1 in 10,000 bp error rate). Draft sequences without base quality values are rejected. Processed sequences are entered into a local database. A version of each sequence with known human repeats masked is also stored. Repeat masking is performed with the program "MASKERAID." Overlap detection: Putative overlaps are detected with the program "WUBLAST." Several filtering steps followed in order to eliminate false overlap detection results, i.e. similarities between a pair of clone sequences that arise due to sequence duplication as opposed to true overlap. Total length of overlap, overall percent similarity, number of sequence differences between nucleotides with high base quality value "high-quality mismatches." Results are also compared to results of restriction fragment mapping of genomic clones at Washington University Genome Sequencing Center, finisher's reports on overlaps, and results of the sequence contig building effort at the NCBI. SNP detection: Overlapping pairs of clone sequence are analyzed for candidate SNP sites with the 'POLYBAYES' SNP detection software. Sequence differences between the pair of sequences are scored for the probability of representing true sequence variation as opposed to sequencing error. This process requires the presence of base quality values for both sequences. High-scoring candidates are extracted. The search is restricted to substitution-type single base pair variations. Confidence score of candidate SNP is computed by the POLYBAYES software.

In method identified by KWOK (TaqMan assay), the TaqMan assay is used to determine genotypes for 90 random individuals. In method identified by KYUGEN(Q1), DNA samples of indicated populations are pooled and analyzed by PLACE-SSCP. Peak heights of each allele in the pooled analysis are corrected by those in a heterozygote, and are subsequently used for calculation of allele frequencies. Allele frequencies higher than 10% are reliably quantified by this method. Allele frequency = 0 (zero) means that the allele was found among individuals, but the corresponding peak is not seen in the examination of pool. Allele frequency = 0-0.1 indicates that minor alleles are detected in the pool but the peaks are too low to reliably quantify.

In yet another method identified as KYUGEN (Method1), PCR products are post-labeled with fluorescent dyes and analyzed by an automated capillary electrophoresis system under SSCP conditions (PLACE-SSCP). Four or more individual DNAs are analyzed with or without two pooled DNA (Japanese pool and CEPH parents pool) in a series of experiments. Alleles are identified by visual inspection. Individual DNAs with different genotypes are sequenced and SNPs identified. Allele frequencies are estimated from peak heights in the pooled samples after correction of signal bias using peak heights in heterozygotes. For the PCR primers are tagged to have 5'-ATT or 5'-GTT at their ends for post-labeling of both strands. Samples of DNA (10 ng/ul) are amplified in reaction mixtures containing the buffer (10mM Tris-HCl, pH 8.3 or 9.3, 50mM KCl, 2.0mM MgCl₂), 0.25µM of each primer, 200µM of each dNTP, and 0.025 units/µl of Taq DNA polymerase premixed with anti-Taq antibody. The two strands of PCR products are differentially labeled with nucleotides modified with R110 and R6G by an exchange reaction of Klenow fragment of DNA polymerase I. The reaction is stopped by adding EDTA, and unincorporated nucleotides are dephosphorylated by adding calf intestinal alkaline phosphatase. For the SSCP: an aliquot of fluorescently labeled PCR products and TAMRA-labeled internal markers are added to deionized formamide, and denatured. Electrophoresis is performed in a capillary using an ABI Prism 310 Genetic Analyzer. Genescan softwares (P-E Biosystems) are used for data collection and data processing. DNA of individuals (two to eleven) including those who showed different genotypes on SSCP are subjected for direct sequencing using big-dye terminator chemistry, on ABI Prism 310 sequencers. Multiple sequence trace files obtained from ABI Prism 310 are processed and aligned by Phred/Phrap and viewed using Consed viewer. SNPs are identified by PolyPhred software and visual inspection.

In yet another method identified as KYUGEN (Method2), individuals with different genotypes are searched by denaturing HPLC (DHPLC) or PLACE-SSCP (Inazuka *et al.,* 1997) and their sequences are determined to identify SNPs. PCR is performed with primers tagged with 5'-ATT or 5'-GTT at their ends for post-labeling of both strands. DHPLC analysis is carried out using the WAVE DNA fragment analysis system (Transgenomic). PCR products are injected into DNASep column, and separated under the conditions determined using WAVEMaker program (Transgenomic). The two strands of PCR products that are differentially labeled with nucleotides modified with R110 and R6G by an exchange reaction of Klenow fragment of DNA polymerase I. The reaction is stopped by adding EDTA, and unincorporated nucleotides are dephosphorylated by adding calf intestinal alkaline phosphatase. SSCP followed by electrophoresis is performed in a capillary using an ABI Prism 310 Genetic Analyzer. Genescan softwares (P-E Biosystems). DNA of individuals including those who showed different genotypes on DHPLC or SSCP are subjected for direct sequencing using big-dye terminator chemistry, on ABI Prism 310 sequencer. Multiple sequence trace files obtained from ABI Prism 310 are processed and aligned by Phred/Phrap and viewed using Consed viewer. SNPs are identified by PolyPhred software and visual inspection. Trace chromatogram data of EST sequences in Unigene are processed with PHRED. To identify likely SNPs, single base mismatches are reported from multiple sequence alignments produced by the programs PHRAP, BRO and POA for each Unigene cluster. BRO corrected possible misreported EST orientations, while POA identified and analyzed non-linear alignment structures indicative of gene mixing/chimeras that might produce spurious SNPs. Bayesian inference is used to weigh evidence for true polymorphism versus sequencing error, misalignment or ambiguity, misclustering or chimeric EST sequences, assessing data such as raw chromatogram height, sharpness, overlap and spacing; sequencing error rates; context-sensitivity; cDNA library origin, etc.

### XIII. Pharmaceutical Formulations and Routes of Administration

The therapeutic compounds of the present disclosure may be administered by a variety of methods, *e.g.*, orally or by injection (*e.g.* subcutaneous, intravenous, intraperitoneal, *etc.*). Depending on the route of administration, the active compounds may be coated in a material to protect the compound from the action of acids and other natural conditions which may inactivate the compound. They may also be administered by continuous perfusion/infusion of a disease or wound site.

To administer the therapeutic compound by other than parenteral administration, it may be necessary to coat the compound with, or co-administer the compound with, a material. to prevent its inactivation. For example, the therapeutic compound may be administered to a patient in an appropriate carrier, for example, liposomes, or a diluent. Pharmaceutically acceptable diluents include saline and aqueous buffer solutions. Liposomes include water-in-oil-in-water CGF emulsions as well as conventional liposomes (Strejan *et al.,* 1984).

The therapeutic compound may also be administered parenterally, intraperitoneally, intraspinally, or intracerebrally. Dispersions can be prepared in glycerol, liquid polyethylene glycols, and mixtures thereof and in oils. Under ordinary conditions of storage and use, these preparations may contain a preservative to prevent the growth of microorganisms.

Pharmaceutical compositions suitable for injectable use include sterile aqueous solutions (where water soluble) or dispersions and sterile powders for the extemporaneous preparation of sterile injectable solutions or dispersion. In all cases, the composition must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (such as, glycerol, propylene glycol, and liquid polyethylene glycol, and the like), suitable mixtures thereof, and vegetable oils. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, sodium chloride, or polyalcohols such as mannitol and sorbitol, in the composition. Prolonged absorption of the injectable compositions can be brought about by including in the composition an agent which delays absorption, for example, aluminum monostearate or gelatin.

Sterile injectable solutions can be prepared by incorporating the therapeutic compound in the required amount in an appropriate solvent with one or a combination of ingredients enumerated above, as required, followed by filtered sterilization. Generally, dispersions are prepared by incorporating the therapeutic compound into a sterile carrier which contains a basic dispersion medium and the required other ingredients from those enumerated above. In the case of sterile powders for the preparation of sterile injectable solutions, the preferred methods of preparation are vacuum drying and freeze-drying which yields a powder of the active ingredient (*i.e.,* the therapeutic compound) plus any additional desired ingredient from a previously sterile-filtered solution thereof.

The therapeutic compound can be orally administered, for example, with an inert diluent or an assimilable edible carrier. The therapeutic compound and other ingredients may also be enclosed in a hard or soft shell gelatin capsule, compressed into tablets, or incorporated directly into the subject's diet. For oral therapeutic administration, the therapeutic compound may be incorporated with excipients and used in the form of ingestible tablets, buccal tablets, troches, capsules, elixirs, suspensions, syrups, wafers, and the like. The percentage of the therapeutic compound in the compositions and preparations may, of course, be varied. The amount of the therapeutic compound in such therapeutically useful compositions is such that a suitable dosage will be obtained.

It is especially advantageous to formulate parenteral compositions in dosage unit form for ease of administration and uniformity of dosage. Dosage unit form as used herein refers to physically discrete units suited as unitary dosages for the subjects to be treated; each unit containing a predetermined quantity of therapeutic compound calculated to produce the desired therapeutic effect in association with the required pharmaceutical carrier. The specification for the dosage unit forms of the invention are dictated by and directly dependent on (a) the unique characteristics of the therapeutic compound and the particular therapeutic effect to be achieved, and (b) the limitations inherent in the art of compounding such a therapeutic compound for the treatment of a selected condition in a patient.

The therapeutic compound may also be administered topically to the skin, eye, or mucosa. Alternatively, if local delivery to the lungs is desired the therapeutic compound may be administered by inhalation in a dry-powder or aerosol formulation.

Active compounds are administered at a therapeutically effective dosage sufficient to treat a condition associated with a condition in a patient. For example, the efficacy of a compound can be evaluated in an animal model system that may be predictive of efficacy in treating the disease in humans, such as the model systems shown in the examples and drawings.

The actual dosage amount of a compound of the present disclosure or composition comprising a compound of the present disclosure administered to a subject may be determined by physical and physiological factors such as age, sex, body weight, severity of condition, the type of disease being treated, previous or concurrent therapeutic interventions, idiopathy of the subject and on the route of administration. These factors may be determined by a skilled artisan. The practitioner responsible for administration will typically determine the concentration of active ingredient(s) in a composition and appropriate dose(s) for the individual subject. The dosage may be adjusted by the individual physician in the event of any complication.

An effective amount typically will vary from about 0.001 mg/kg to about 1000 mg/kg, from about 0.01 mg/kg to about 750 mg/kg, from about 100 mg/kg to about 500 mg/kg, from about 1.0 mg/kg to about 250 mg/kg, from about 10.0 mg/kg to about 150 mg/kg in one or more dose administrations daily, for one or several days (depending of course of the mode of administration and the factors discussed above). Other suitable dose ranges include 1 mg to 10000 mg per day, 100 mg to 10000 mg per day, 500 mg to 10000 mg per day, and 500 mg to 1000 mg per day. In some particular embodiments, the amount is less than 10,000 mg per day with a range of 750 mg to 9000 mg per day.

The effective amount may be less than 1 mg/kg/day, less than 500 mg/kg/day, less than 250 mg/kg/day, less than 100 mg/kg/day, less than 50 mg/kg/day, less than 25 mg/kg/day or less than 10 mg/kg/day. It may alternatively be in the range of 1 mg/kg/day to 200 mg/kg/day. For example, regarding treatment of diabetic patients, the unit dosage may be an amount that reduces blood glucose by at least 40% as compared to an untreated subject. In another embodiment, the unit dosage is an amount that reduces blood glucose to a level that is ± 10% of the blood glucose level of a non-diabetic subject.

In other non-limiting examples, a dose may also comprise from about 1 microgram/kg/body weight, about 5 microgram/kg/body weight, about 10 microgram/kg/body weight, about 50 microgram/kg/body weight, about 100 microgram/kg/body weight, about 200 microgram/kg/body weight, about 350 microgram/kg/body weight, about 500 microgram/kg/body weight, about 1 milligram/kg/body weight, about 5 milligram/kg/body weight, about 10 milligram/kg/body weight, about 50 milligram/kg/body weight, about 100 milligram/kg/body weight, about 200 milligram/kg/body weight, about 350 milligram/kg/body weight, about 500 milligram/kg/body weight, to about 1000 mg/kg/body weight or more per administration, and any range derivable therein. In non-limiting examples of a derivable range from the numbers listed herein, a range of about 5 mg/kg/body weight to about 100 mg/kg/body weight, about 5 microgram/kg/body weight to about 500 milligram/kg/body weight, *etc.*, can be administered, based on the numbers described above.

In certain embodiments, a pharmaceutical composition of the present disclosure may comprise, for example, at least about 0.1% of a compound of the present disclosure. In other embodiments, the compound of the present disclosure may comprise between about 2% to about 75% of the weight of the unit, or between about 25% to about 60%, for example, and any range derivable therein.

Single or multiple doses of the agents are contemplated. Desired time intervals for delivery of multiple doses can be determined by one of ordinary skill in the art employing no more than routine experimentation. As an example, subjects may be administered two doses daily at approximately 12 hour intervals. In some embodiments, the agent is administered once a day.

The agent(s) may be administered on a routine schedule. As used herein a routine schedule refers to a predetermined designated period of time. The routine schedule may encompass periods of time which are identical or which differ in length, as long as the schedule is predetermined. For instance, the routine schedule may involve administration twice a day, every day, every two days, every three days, every four days, every five days, every six days, a weekly basis, a monthly basis or any set number of days or weeks therebetween. Alternatively, the predetermined routine schedule may involve administration on a twice daily basis for the first week, followed by a daily basis for several months, *etc.* In other embodiments, the invention provides that the agent(s) may taken orally and that the timing of which is or is not dependent upon food intake. Thus, for example, the agent can be taken every morning and/or every evening, regardless of when the subject has eaten or will eat.

### XIV. Combination Therapy

Effective combination therapy may be achieved with a single composition or pharmacological formulation that includes both agents, or with two distinct compositions or formulations, administered at the same time, wherein one composition includes a compound of this invention, and the other includes the second agent(s). Alternatively, the therapy may precede or follow the other agent treatment by intervals ranging from minutes to months.

Various combinations may be employed, such as where "A" represents the first agent (*e.g.,* DFMO) and "B" represents a secondary agent (*e.g.,* sulindac), non-limiting examples of which are described below:

| | | | | | |
|---|---|---|---|---|---|
| A/B/A | B/A/B B/B/A | A/A/B | A/B/B B/A/A | A/B/B/B | B/A/B/B |
| B/B/B/A | B/B/A/B | A/A/B/B | A/B/A/B | A/B/B/A | B/B/A/A |
| B/A/B/A | B/A/A/B | A/A/A/B | B/A/A/A | A/B/A/A | A/A/B/A |

### XV. Examples

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice.

### Reference Example 1 - Epidemiologic Studies: ODC +316 SNP Associations with CRC-Specific Survival

*Experimental Design:* The study included 440 incident CRC cases from the population-based UC Irvine Gene-Environment Study of Familial CRC (diagnosed 1994-1996 with follow-up through March 2008). The primary outcome was CRC-specific survival (CRC-SS) dependent on ODC genotype (GG vs. AA/GA). In human colon cancer cell lines, ODC allele-specific binding of E-box transcription factors was determined via western blotting and chromatin immunoprecipitation (CHIP) assays. ODC allele-specific promoter activity was determined using promoter constructs in combination with vectors expressing either the transcriptional activator *c-MYC* or the repressor *MAD1.*

*Results:* Genotype-specific survival differences among CRC cases were limited to colon cancer cases: compared to ODC GG genotype cases (HR=1.00, reference) the adjusted CRC-SS hazards ratio (HR) was 2.31 (1.15-4.64) for ODC GA cases and 3.73 (0.93-14.99) for ODC AA cases (P-trend=0.006). In colon cancer cells, the ODC +316 SNP, flanked by two E-boxes, predicts ODC promoter activity. The E-box activator *-MYC* and repressors *MAD1* and MAD4 preferentially bind to minor A-, compared to major G-, alleles in cultured cells.

***Study population**.* We studied incident cases of invasive CRC enrolled in the University of California, Irvine Gene-Environment Study of Familial Colorectal Cancer (Peel *et al.,* 2000; Zell *et al.,* 2007) during 1994-1996 with follow-up through March 2008. The parent study was designed to determine the incidence of HNPCC in a large, population-based cohort of colorectal cancer cases. Participants were identified through the population-based cancer registries of the Cancer Surveillance Program of Orange County/San Diego Imperial Organization for Cancer Control using the April 2008 data file. In the parent study (Peel *et al.,* 2000), all subjects with CRC diagnosed at all ages in Orange County, CA, from 1994 to1996 were ascertained. All subjects diagnosed in San Diego and Imperial Counties, CA, at ages <65 y between 1994 and 1995 were also ascertained. Cases were then contacted if they were eligible for the study (alive at the time ascertained and having a contact address) and if their physicians did not deny permission to contact. At the time of study entry, cases signed a consent form allowing for blood draws and the release of medical information. This study was approved by the UC Irvine Institutional Review Board (#93-257). Clinical and demographic data including vital status and follow-up were obtained through linkage to the regional cancer registry databases as previously described (Peel *et al.,* 2000; Zell *et al.,* 2007; Zell *et al.,* 2008). Tumor, node, metastasis (TNM) staging determination was derived from existing AJCC codes where available and conversion of extent of disease codes, as previously reported (Le *et al.,* 2008). Family history of cancer in a first-degree relative was ascertained by self-reporting during a telephone interview conducted at enrollment (Zell *et al.,* 2008; Ziogas and Anton-Culver, 2003). Twenty-two cases with hereditary non-polyposis colon cancer (HNPCC), as defined by Amsterdam criteria, were identified and excluded from the analysis. The median time from CRC diagnosis until study entry (i.e., date of family history interview) was 16 months (95% CI 12-23 months).

***DNA extraction and ODC +316 SNP genotyping.*** DNA was extracted from 2.0 mL red blood cell clot samples using the QIAGEN QIAamp DNA Midi or Mini Kits, (Qiagen) following the manufacturer's instructions. Genotyping of the ODC +316 SNP was conducted using oligonucleotide primers designed to amplify a 172-bp fragment containing the polymorphic base at +316 (Applied Biosystems, Foster City, CA). Allele-specific TaqMan probes were synthesized with different 5' labels (6-carboxyflourescein or VIC) and the same 3' quencher dye (6-carboxytetramethylrhodamine) (23). Each PCR reaction (5 µL total) contained 10 ng of participant DNA, 30 pmol of each primer, 12.5 pmol of each TaqMan probe, and 1x TaqMan Universal PCR Master Mix (Applied Biosystems, Foster City, CA), as previously reported (Martinez *et al.,* 2003; Guo *et al.,* 2000).

***Statistical analysis* -** ***population-based study.*** Sample size was determined based on an estimated 1:1 ratio of ODC GG genotype to ODC GA/AA genotype (Martinez *et al.,* 2003; Barry *et al.* 2006; Hubner *et al.,* 2008; Guo *et al.,* 2000). Prior analysis of data from 1154 colon and rectal cancer cases in the UC Irvine Gene-Environment Study of Familial CRC revealed that 10-year CRC-specific survival approximated 66% (Zell *et al.,* 2008). The inventors proposed a 15% or greater difference in CRC-specific survival based on ODC genotype alone for our sample size calculations. Thus, 315 total subjects were needed to detect the proposed difference in 10-year CRC-specific survival between two groups at 5% significance level with 80% power: 55% in group 1 vs. 70% in group 2. 440 of 481 DNA samples were successfully genotyped. 41 cases (8.5%) resulted in an undetermined ODC +316 genotype due to low DNA concentration and/or poor DNA quality, however no clinicopathologic differences were observed between the successfully genotyped and unsuccessfully genotyped cases. Thus the study was sufficiently powered to address the primary endpoint.

Comparisons of demographic, clinical, and pathologic variables among colon and rectal cases were done using Pearson chi-square statistic or Fisher's exact test for nominal variables and Student t-test for continuous variables. Colorectal cancer-specific survival was defined as mortality from CRC itself, and data censoring occurred in the following instances: alive at the end of follow-up, loss to follow-up, or death from any cause other than CRC. Overall survival (OS) was defined with mortality from any cause. Survival curves were constructed for colon and rectal cancer cases using the Kaplan-Meier method and analyzed with the log rank test for univariate analyses. Cox proportional hazards modeling was performed for all CRC cases, colon cancer cases, and rectal cancer cases using time since diagnosis to profile the adjusted risk of overall and CRC-specific death based on ODC genotype. The effects of ODC genotype (GG, GA, or AA) on survival were analyzed in the Cox models with adjustment for the following covariates: age, gender, ethnicity, family history of CRC, TNM stage at diagnosis, tumor site within the colon, histologic subtype, treatment with surgery, radiation therapy, and chemotherapy. Each variable in the model was coded using dummy variables. All analyses were conducted using SAS 9.2 statistical software (SAS Institute, Cary, NC). Statistical significance was assumed for a 2-tailed P value <0.05.

### Reference Example 2 - Experimental Studies: ODC +316 SNP Regulation in Colon Cancer Cells

***Cell culture.*** The human colon cancer cell lines HT29 and HCT116 were maintained in McCoy's 5A medium (Invitrogen, Carlsbad, CA). All media used were supplemented with 10% FBS plus 1% penicillin/streptomycin solution (Invitrogen, Carlsbad, CA). Cultures were maintained at 37°C in a humidified atmosphere of 5% CO2.

***Genotyping assay.*** DNA samples from HT29 and HCT116 cells were subjected to a PCR-RFLP procedure to detect the polymorphic PstI site. Sequences were amplified by PCR, using the following primers: 5'- TCTGCGCTTCCCCATGGGGCT-3' (SEQ ID NO:1) and 5'-TTTCCCAACCCTTCG-3' (SEQ ID NO:2). Each reaction contained - 1 µl DNA, 4 pmol of each primer, 12.5 µl 2x PCR PreMixes buffer "G" (EPICENTRE Biotechnologies, Madison, WI) and 0.5 unit of Taq DNA polymerase, in a final volume of 25 µl. The expected size of the PCR product was 351 bp. After amplification, 10-20 µl of the PCR product were digested with 10 units of PstI in 30 µl for 2 hours at 37oC. DNA from HT29 cells (GA), containing the PstI site, yielded two fragments of 156 and 195 bp.

***Western blot analysis.*** Cells were harvested, lysed and proteins were separated on a 12.5% SDS-PAGE gel. Proteins were transferred by electrophoresis onto a Hybond-C membrane. The membrane was blocked with Blotto A (5% blocking grade dry milk in TTBS solution) and probed using 1:300 dilutions of primary antibodies (Santa Cruz Biotechnology, Santa Cruz, CA) in Blotto A. Primary antibodies were incubated at 4°C overnight, followed by incubation with an appropriate HRP-tagged secondary antibody (1:1000 dilution) for 1 hour at room temperature. Chemiluminescent detection was conducted using ECL Western Detection reagent (Amersham Biosciences, Piscataway, NJ) and exposed on Biomax XAR film (Kodak).

***Chromatin immunoprecipitation (CHIP).*** CHIP assays were performed using a commercial kit, as recommended by the manufacturer (Upstate Biotech, Lake Placid, NY, USA). Briefly, cells were treated with 1% formaldehyde to crosslink DNA and proteins, and DNA-protein complexes were disrupted by sonication to lengths between 200 to 1000 bp. Lysates were diluted 10-fold with immunoprecipitation (IP) dilution buffer containing protease inhibitors. Antibodies for *c-MYC, MAD1* and MAD4 (Santa Cruz Biotechnology, Santa Cruz, CA) were used to precipitate chromatin, while additional sample was left as a minus-antibody (-Ab) control. Samples were immunoprecipitated overnight at 4oC with rotation. Immune complexes were obtained by adding 60ul of salmon sperm DNA/protein A Agarose slurry and incubating for an hour at 4oC with rotation followed by gentle centrifugation (1000 rpm, 1 min). Protein A agarose pellets were washed with low salt buffer, high salt buffer, LiCl buffer and TE buffer. Then the complexes were eluted by adding 250 µl elution buffer (0.1M NaHCO3, 1% SDS) twice, and DNA-protein crosslinks were reversed with 0.2 M NaCl by heating at 65oC for 4 hours for all samples, including the input DNA and -Ab DNA controls. DNA was resuspended in 30ul of ddH2O. For visualization of PCR product and its size, standard PCR reactions were carried out. The sequences of ODC primers used for PCR were 5'- CCTGGGCGCTCTGAGGT-3' (SEQ ID NO:3) (17mer) and 5'-AGGAAGCGGCGCCTCAA-3' (SEQ ID NO:4) (17mer). Quantitative real-time PCR was performed using TaqMan gene expression assays kit (Applied Biosystems, Foster City, CA) on an ABI7700 sequence detection system. Details for the computation of relative binding can be found on the manufacturer's web site (http://www.appliedbiosystems.com/).

***Transient transfections**.* Transient transfections were preformed using LipofectAMINE reagent (Invitrogen, Carlsbad, CA) according to the manufacturer's protocol, as detailed in the supplementary file. HCT116 and HT29 cells were transfected with 1 µg of pGL3-ODC/A or pGL3-ODC/G plasmids (Martinez *et al.,* 2003) along with 0.01 µg of Renilla-TK plasmid. The Renilla-TK plasmid was purchased from Promega (Madison, WI) and used as a transfection efficiency control in all promoter-reporter transfection experiments. For *c-MYC* experiments, ODC pGL3-plasmids were co-transfected with either pcDNA 3.0 or CMV-*c*-*MYC* expression vector (OriGene, Rockville, MD). For *MAD1* experiments, the ODC plasmids were co-transfected with either pcDNA 3.1 or pcDNA-*AMD1*. For *c-MYC* and *MAD1* co-transfection, ODC promoter reporter constructs were prepared which contain the first 1.6 Kb of the ODC gene cloned into a pGL3 vector. The constructs included E-box 1 (-485 to -480 bp) intact (wt E-box 1) or deleted (mut E-box 1). Additionally, both variants of the +316 ODC SNP were used, creating a total of 4 different constructs. After 6 hours of incubation, cells were supplemented with complete medium containing 20% FBS and left to grow overnight. The next day after transfection 20% FBS-containing complete medium was replaced with 10% FBS-containing medium. 48 hours after transfection, cells were washed with PBS and lysed in Passive Lysis Buffer from the Dual Luciferase Assay kit (Promega, Madison, WI). Dual luciferase activities were measured using a Turner Designs TD-20/20 luminometer, as described by the manufacturer, and presented as relative luciferase units (RLU) . Experiments were preformed in triplicates and repeated at least 2 times.

***Statistical analysis* -** ***experimental studies.*** For transient transfection experiments, two-sample t-tests were used (Microsoft Excel Microsoft Corp., Redmond, WA). The effect of *c-MYC* expression on ODC allele-specific promoter activity was examined in HT29 colon cancer cells using ODC promoter constructs differing by the presence of the first E-box element: (a) wild type (wt) E-box1+316 G, (b) mutant (mut) E-box1 + 316 G, (c) wt E-box1 + 316 A, and (d) mut E-box1 + 316 A. For each promoter construct, two-sample t-tests were used to compare promoter activity between cells co-transfected with pcDNA3.0 plasmid versus those transfected with the CMV-c-MYC expression vector. Similarly, to examine the effect of *MAD1* expression on ODC allele-specific promoter activity, two-sample t-tests were used to compare the effect of promoter activity in promoter constructs co-transfected with pcDNA3.1 plasmid versus those transfected with pcDNA*-MAD1* plasmid. Statistical significance was assumed for a 2-tailed P value <0.05.

### Reference Example 3 - Differential Affects of ODC1 Genotype

This study involves analysis of patient data from the multicenter phase III colon adenoma prevention trial (Meyskens *et al.,* 2008). 375 patients were enrolled, and the study was halted by the Data Safety Monitoring Board (DSMB) after 267 patients completed end-of-study colonoscopies (due to the study meeting its efficacy endpoints). The DSMB monitored all safety and efficacy endpoints. Blood specimens were collected on 228 consenting study patients for genotyping analysis after November 2002 (including 159 of 246 patients randomized before, and 69 of 129 patients randomized after this date), when the protocol was modified in light of data demonstrating the importance of the *ODC1* SNP (2). *ODC1* (rs2302615) genotyping was conducted on patient-derived genomic DNA using allele-specific TaqMan probes as described previously (Guo *et al.,* 2000). Rectal tissue polyamine content was determined as described previously (Meyskens *et al.,* 1998; Seiler and Knodgen, 1980), using 3 of 8 randomly selected rectal mucosal biopsy specimens. Tissue polyamine response was performed for response values ranging from 25% to 45%.

*ODC1* genotype was analyzed under a dominant model: AA/GA vs. GG patients. Wilcoxon Rank Sums tests were performed on non-normally distributed continuous variables across two genotype groups. Chi-square tests or Fisher's Exact Test were utilized to assess the association between baseline categorical variables and genotype group. Log binomial regression was performed on the primary outcome (adenoma recurrence) with predictors: treatment group, age, gender, race (Caucasian vs. other), aspirin usage, *ODC1* genotype (in the dominant model), and a term representing the treatment by genotype interaction. For secondary outcomes (rectal tissue polyamine response, toxicities), the effects of treatment group, genotype, and interaction between treatment and genotype were examined using full log binomial models. Statistical analyses were conducted using SAS 9.2 statistical software (SAS Inc., Cary, NC). Patients signed informed consent for trial inclusion and specimen retrieval/analysis. The study was approved after full committee review by the UC Irvine institutional review board (IRB protocol #2002-2261) and review by each of the local IRBs at participating study sites.

*ODC1* genotype distribution was: 126 GG (55%), 87 GA (38%), and 15 AA (7%). Baseline clinical characteristics revealed differences, as shown in Table 1. The relative risk (RR) for adenoma recurrence related to treatment after adjustment in the full regression model was 0.39 (95% CI 0.24-0.66). Among patients receiving placebo or treatment, respectively, ototoxicity occurred in 23% vs. 22% of *ODC1* GG patients, 20% vs. 21% of *ODC1* GA patients, and 0% (0 of 7) vs. 57% (4 of 7) of *ODC1* AA patients.

### Example 4 - Dietary Polyamine Intake Analysis

***Patient Population, Description of Parent Study.*** This study involves analysis of patient data and specimens from the multicenter colon adenoma prevention trial, as described elsewhere. Meyskens FL, McLaren C.E. , Pelot D., Fujikawa S., et al: Difluoromethylornithine plus sulindac for the prevention of sporadic colorectal adenomas: a randomized placebo-controlled, double-blind trial. Cancer Prevention Research 1:32-38, 2008. It originated as a randomized double-blind, placebo-controlled phase IIb trial of DFMO 500 mg daily in combination with sulindac 150 mg daily *versus* placebo in patients with prior history of colorectal adenoma, and was later expanded in 2003 to a phase III clinical trial. Three hundred seventy-five patients were randomized to receive treatment with either DFMO and sulindac, or placebo. Stratification was performed for study site and prior low-dose aspirin usage. Planned treatment duration was 36 months. Baseline and end-of study endoscopies were performed, each with procurement of eight rectal mucosa tissue biopsies. Clinical data were collected at baseline interview and recorded in the study chart. A food frequency questionnaire completed by the patients was collected at the initiation of the study. At the second interim analysis, the study was halted since the clinical efficacy endpoints were achieved; thus 267 patients completed the trial. Meyskens FL, McLaren C.E. , Pelot D., Fujikawa S., et al.: Difluoromethylornithine plus sulindac for the prevention of sporadic colorectal adenomas: a randomized placebo-controlled, double-blind trial. Cancer Prevention Research 1:32-38, 2008.

***Dietary Polyamine Intake**.* Dietary intakes of participants in this clinical trial were estimated with the Fred Hutchinson Cancer Research Center food frequency questionnaire (FFQ), and the analytic algorithms for this instrument are published elsewhere. Kristal AR, Shattock, A. L., and Williams, A. E.: Food frequency questionnaires for diet intervention research. Washington, DC: International Life Sciences Institute,. Proceeding of the 17th National Nutrient Databank Conference, Baltimore, MD, 1992., 1992; Schakel SF, Buzzard, I M., and Gebhardt, S. E. : Procedures for estimating nutrient values for food composition databases.. J Food Comp Anal 10: 102-14, 1997 To estimate dietary polyamines in the 370 foods listed in this FFQ, a polyamine food content database was developed and linked to the FFQ, for which the University of Minnesota Nutrition Coordinating Center (NCC) Nutrient Database serves as the primary source of food content data, as described by Zoumas-Morse *et al.* Zoumas-Morse C, Rock CL, Quintana EL, et al: Development of a polyamine database for assessing dietary intake. J Am Diet Assoc 107:1024-7, 2007. Values for spermine, spermidine and putrescine in individual food items were calculated and expressed as nmol/g. Dietary putrescine was the major contributor to total dietary polyamines intake. Total daily dietary polyamine content was derived by adding the dietary putrescine, spermine and spermidine and further categorized into highest (75-100%) quartile group *versus* a group with lower three quartiles (0-25, 25-50 and 50-75%). The results were energy-adjusted in the analysis.

***Tissue Polyamine Analysis.*** Rectal tissue polyamine content was determined as previously described. Meyskens FL, Gerner EW, Emerson S, et al: Effect of alpha-difluoromethylornithine on Rectal Mucosal levels of polyamines in a randomized, double-blinded trial for colon cancer prevention. Journal of the National Cancer Institute 90:1212-1218, 1998; Boyle JO, Meyskens FL, Jr., Garewal HS, et al.: Polyamine contents in rectal and buccal mucosae in humans treated with oral difluoromethylornithine. Cancer Epidemiol Biomarkers Prev 1:131-5, 1992. Polyamine content was evaluated using 3 of 8 randomly selected rectal mucosal biopsy specimens. These methods measured putrescine, cadaverine, histamine, spermidine, spermine, and monoacetyl derivatives of putrescine, spermidine, and spermine. Tumor-free rectal biopsies were collected, flushed with ice-cold saline, and stored frozen at -80°C. Samples were processed, then assayed for polyamine content by reverse-phase high performance liquid chromatography with 1,7-diaminoheptane as an internal standard. Seiler N, Knodgen B: High-performance liquid chromatographic procedure for the simultaneous determination of the natural polyamines and their monoacetyl derivatives. J.Chromatogr. 221:227-235, 1980. Protein content in each sample was determined using the BCA protein assay kit (Pierce, Rockford, IL). Data were expressed as nmol polyamine per milligram protein.

***Statistical Analysis.*** Descriptive analysis of variables such as age, gender, ethnicity, prior aspirin use, and polyp location, size and histology were performed. A variable called advanced adenoma was created that takes into account polyp size and histological grade, such that any adenoma with villous histology or size > 1 cm was classified as an advanced adenoma. Since dietary and tissue polyamine data were not normally distributed, the non parametric Wilcoxon Rank Sum test was used for comparisons of numeric data in the highest *versus* lower dietary polyamine intake groups. The Chi-square test for independence was used for comparisons of nominal data in the highest *versus* lower dietary polyamine intake groups. Spearman's rank correlation coefficient (*r*ₛ) was used to assess the relationship between dietary polyamine intake at baseline and intakes of protein, animal-derived protein, and arginine. A logistic regression model was used to calculate the recurrence risk of metachronous adenomas. Logistic regression was used to estimate the risk of recurrence of metachronous adenomas. A model was formed with presence of metachronous adenoma as the outcome variable, and predictors including dietary polyamine group, treatment with DFMO + sulindac *(versus* placebo), low-dose aspirin use *(versus* none), and a term representing interaction between dietary polyamine group and treatment. All statistical analyses were conducted using SAS 9.2 statistical software (SAS Inc., Cary, NC).

***Ethical Considerations.*** This study involved analysis of data and tissue from a phase III colorectal adenoma prevention parent trial. Meyskens FL, McLaren C.E. , Pelot D., Fujikawa S., et al.: Difluoromethylornithine plus sulindac for the prevention of sporadic colorectal adenomas: a randomized placebo-controlled, double-blind trial. Cancer Prevention Research 1:32-38, 2008. The parent study was approved after full committee review by the UC Irvine institutional review board (IRB protocol #2002-2261) and each of the local IRBs from the participating clinical study sites. All patients signed informed consent for inclusion into the trial, and specimen retrieval/analysis for research purposes.

***Characteristics of the Study Population.*** Dietary polyamine data were available for 222 of 375 baseline study patients, and for 188 of 267 patients completing the end-of-study colonoscopy. The clinical characteristics of the study population are shown in Table 5. Patient groups were similar in age, gender, race/ethnicity, aspirin use, number of prior polyps, polyp histology and polyp location. Mean age of the study population was 60 years +/- 8 standard deviation (SD), ranging from 47 to 75 years at the time of study entry. The majority of patients were males [164 (74%)] compared with females [58 (26%)]. The majority of patients were white non-Hispanic [192 (86%)], followed by Hispanics [12 (5%)], African-Americans [8 (4%)], and Asian-Americans [7 (3%)]. Prior low-dose aspirin use was reported by 93 patients (42%), compared with 129 non-aspirin users (58%). Ninety-three patients (42%) had just one adenoma at baseline, and the total number of baseline polyps ranged from 1 to 16. The following polyp types were reported: 170 tubular adenomas (77%), 30 tubulovillous adenomas (13%), 13 adenoma-not specified (6%), 6 villous adenomas (3%), 2 carcinoma in-situ (<1%), and 1 adenoma with high-grade dysplasia (<1%). Polyp location was reported as follows: rectum (n = 43, 19%), left colon (n = 87, 39%), transverse colon (31, 14%), right colon (35, 16%), and cecum (26, 12%).

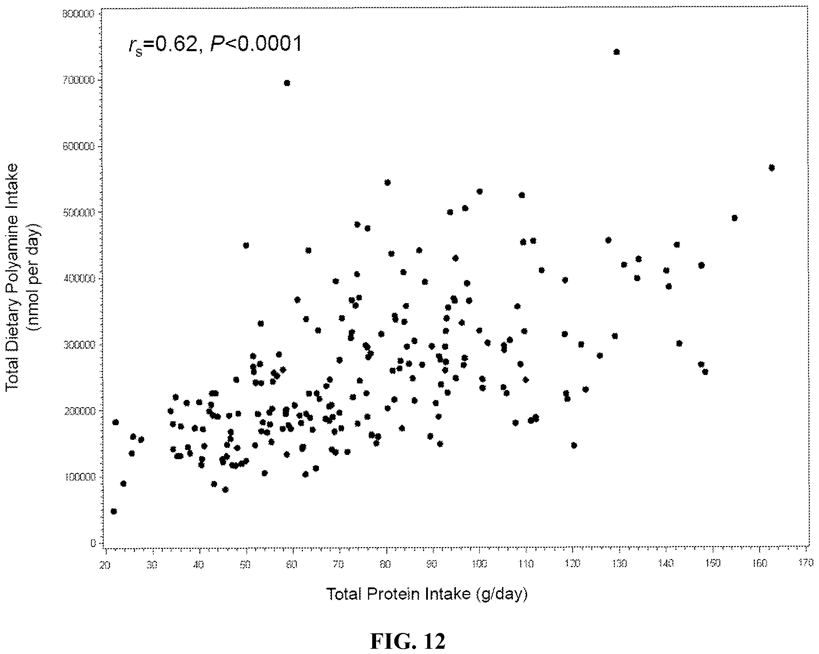

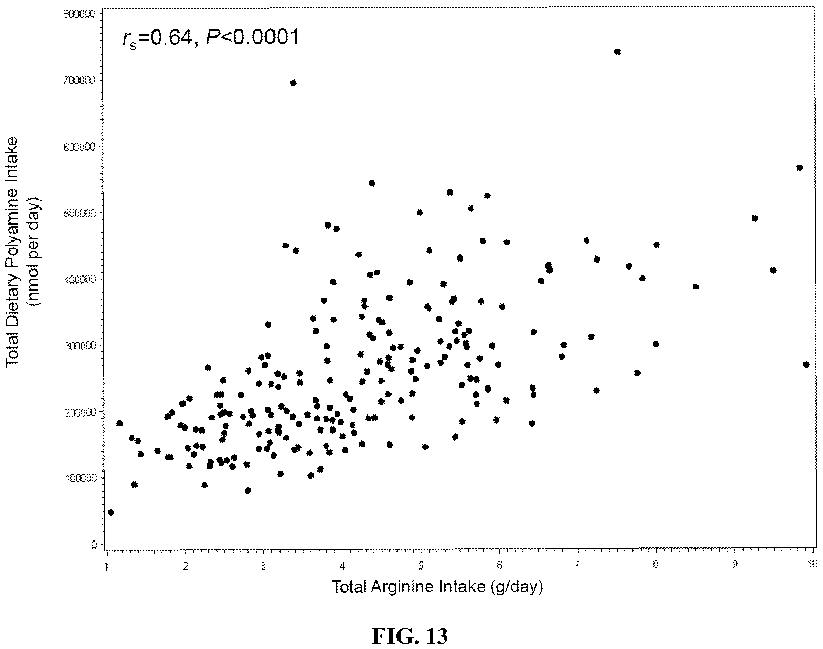

***Dietary polyamines and selected clinical characteristics at baseline.*** The median total daily dietary polyamine intake was 233,261 nmol/day (range 48,692-740,446). Patients in the highest quartile were those reporting more than 318,016 nmol polyamines per day (all others were classified as the lower polyamine intake group). Median total daily intakes of protein (from all sources), animal-derived protein, and arginine were 72.8 g, 51.8 g, and 4.0 g, respectively. At baseline, total daily dietary polyamine intake was correlated with total daily protein intake (*r*ₛ = 0.62, *P* < 0.0001; FIG. 12), total daily animal-derived protein intake (*r*ₛ = 0.49, *P* < 0.0001), and total daily arginine intake (*r*ₛ = 0.64, *P* < 0.0001; FIG. 13). Dietary arginine intake was highly correlated with the individual dietary polyamine components spermine (*r*ₛ = 0.90, *P* < 0.0001) and spermidine (*r*ₛ = 0.74, *P* < 0.0001), and to a lesser degree, dietary putrescine (*r*ₛ = 0.44, *P* < 0.0001).

The mean number of polyps at baseline in the lower dietary polyamine group was 2.61 (± 2.32 SD) compared to 2.41 (± 1.90 SD) in the highest dietary polyamine group, which was not significant (P = 0.98). At baseline, the highest dietary polyamine group had a greater proportion of adenomas larger than 1 cm (43.6 % *versus* 26.4 %; *P* = 0.016), higher grade adenomas (32.7% *versus* 20.4% *P* = 0.060) and a higher proportion with advanced adenomas (52.7 % *versus* 35.9 %; *P* = 0.028). Left sided adenomas *(versus* right) were more common among patients within the lower (61.1%) *versus* the highest dietary polyamine group (49%), but this difference was not statistically significant (Table 6).

***Dietary polyamine intake and rectal tissue polyamines.*** At baseline, mean rectal tissue spermidine and spermine levels were lower among patients in the lower dietary polyamine group (Table 3). Mean tissue spermidine levels were 2.49 *versus* 2.95 nmol/mg (*P* = 0.024), and mean spermine levels were 7.90 *versus* 8.92 nmol/mg (P = 0.039) for patients in the lower and highest dietary polyamine groups, respectively. There were no statistically significant differences in tissue levels of putrescine or the spermidine:spermine ratio across the two dietary polyamines groups (Table 7).

***Dietary polyamines and the effect of treatment on polyp recurrence.*** Our primary aim of the study was to analyze the interaction between dietary polyamines and treatment with DFMO+sulindac in adenoma recurrence. Analysis was done on 188 patients with available dietary polyamine intake data and complete trial data with end-of-study colonoscopy results to assess effects against polyp recurrence. In the logistic regression model for metachronous polyp recurrence, treatment group (DFMO + sulindac *versus* placebo), aspirin use, and dietary polyamine intake group were used as predictors, along with a term representing the interaction between dietary polyamine group and treatment. Among all patients, a significant interaction was observed between treatment with DFMO + sulindac and dietary polyamine groups with the risk of metachronous adenomas (*P* = 0.01). Risk estimates were similar for patients across the lower three dietary polyamine quartiles, therefore the final analysis (n = 188) considered two polyamine intake groups. The inventors observed a significant risk reduction of metachronous adenomas from treatment with DFMO + sulindac *(versus* placebo) in the lower dietary polyamine group [RR, 0.19; 95% CI 0.09-0.40; *P* < 0.0001]; however no difference was observed for treatment *versus* placebo in the highest dietary polyamine group [RR, 1.04; 95% CI, 0.32-3.36; *P* = 0.94]. In the lower dietary polyamine group, significant risk reduction was observed for DFMO + sulindac treatment *(versus* placebo) for the following endpoints: large adenomas [RR, 0.11; 95% CI, 0.01-0.88; *P* = 0.03], high grade adenomas [RR, 0.09; 95% CI, 0.01-0.71; *P* = 0.02], and advanced adenomas [RR, 0.06; 95% CI, 0.008-0.44; P=0.005] (Table 8).

**Table 1. Clinical Characteristics of all Subjects at Baseline (n=228) by ODC1 Genotype.**

| | *ODC1* AA/GA genotype (n=102) | *ODC1* GG genotype (n=126) | *P** |
|---|---|---|---|
| Mean Age (years ± Standard Deviation) | 60.2 ± 8.4 SD | 62.6 ± 8.7 SD | 0.024^{†} |
| Gender (n, %) | | | |
| Male | 77 (75%) | 96 (76%) | 0.90 |
| Female | 25 (25%) | 30 (24%) | |
| Race (n, %) | | | |
| White | 84 (82%) | 107 (85%) | 0.007^{‡} |
| Black | 3 (3%) | 4 (3%) | |
| Hispanic | 4 (4%) | 12 (10%) | |
| Asian | 9 (9%) | 1 (1%) | |
| Other | 2 (2%) | 2 (2%) | |
| Treatment group (n, %) | | | |
| Eflornithine + sulindac | 46 (45%) | 71 (56%) | 0.09 |
| Placebo | 56 (55%) | 55 (44%) | |
| Low-dose aspirin use (n, %) | | | |
| Yes | 44 (43%) | 54 (43%) | 0.97 |
| No | 58 (57%) | 72 (57%) | |
| Median no. (with minimum-maximum) | 2.00 (1,11) | 2.00 (1,16) | 0.41^{†} |
| Location of largest prior polyp (n, %) | | | |
| Rectum | 26 (25%) | 23 (18%) | 0.19 |
| Colon | 76 (75%) | 103 (82%) | |
| Prior polyp histology (n, %) | | | |
| Tubular | 76 (75%) | 99 (79%) | 0.03^{‡} |
| Adenoma-NOS | 6 (6%) | 8 (6%) | |
| Tubulovillous | 10 (10%) | 17 (13%) | |
| Villous | 7 (7%) | 1 (1%) | |
| Carcinoma in-situ | 3 (3%) | 0 (0%) | |
| Tubular adenoma, high-grade | 0 (0%) | 1 (1%) | |
| dysplasia | | | |
| Largest polyp ≥1 cm (n, %) | 25 (25%) | 40 (32%) | 0.23 |
| Treatment rendered for prior polyp (n, %) | 92(90%) | 117(93%) | 0.47 |
| Complete endoscopic removal | 10 (10%) | 9 (7%) | |
| Surgery | | | |
| Baseline tissue polyamine content^{§} (median, nmol/mg protein , range) | | | |
| Putrescine | 0.47 (0.01-4.60) | 0.56 (0.01-5.29) | 0.48^{†} |
| Spermidine | 1.99 (0.76-9.18) | 2.17 (1.05-8.97) | 0.08^{†} |
| Spermine | 6.82 (2.29-19.86) | 7.29 (2.72-22.85) | 0.23^{†} |
| Spermidine:Sermine ratio | 0.30 (0.19-0.98) | 0.31 (0.19-0.76) | 0.23^{†} |

| | | | |
|---|---|---|---|
| *p-value for the X² test is listed unless noted otherwise. ^{†}p-value for the Wilcoxon Rank Sums test. ^{‡}p-value for the Fisher Exact test. ^{§}Tissue polyamine data missing for 1 subject with *ODC1* GG genotype and 1 subject with *ODC1* AA/GA genotype | | | |

**Table 2. Multivariate Overall Survival and Colorectal Cancer-Specific Survival Analysis for Colorectal Cancer Cases Based on ODC1 Genotype.**

| | ***ODC1* Genotype** | | ***P*** |
|---|---|---|---|
| | **GG** | **GA/AA** | |
| Overall mortality | | | |
| Number of events | 47 | 62 | |
| Number at risk | 208 | 192 | |
| Unadjusted HR (95% CI) | 1 (reference) | 1.57 (1.07-2.29) | 0.020 |
| Adjusted HR (95% CI)* | 1 (reference) | 1.58 (1.07-2.34) | 0.021 |
| CRC-specific mortality | | | |
| Number of events | 22 | 37 | |
| Number at risk | 208 | 192 | |
| Unadjusted HR (95% CI) | 1 (reference) | 1.97 (1.16-3.34) | 0.012 |
| Adjusted HR (95% CI)* | 1 (reference) | 2.02 (1.17-3.50) | 0.012 |

| | | | |
|---|---|---|---|
| Abbreviation: 95% Cl, 95% confidence interval. *Includes stratification for stage (I, II, III) and adjustment for age (y), gender, ethnicity, family history of colorectal cancer, TNM stage at diagnosis, tumor site within the colorectum, histologic subtype, treatment with surgery, radiation therapy, and chemotherapy. | | | |

**Table 3. Incidence of Events after Randomization and Stratified by ODC1 Genotype (Dominant Model).**

| | **Placebo (n = 111)** | | **Eflornithine/Sulindac (n = 117)** | | ***P**** |
|---|---|---|---|---|---|
| | *ODC1* GG | *ODC1* GA or AA | *ODC1* GG | *ODC1* GA or AA | |
| **Any adenoma recurrence** | 22/44(50) | 18/53(34) | 7/64(11) | 9/42(21) | < 0.0001 |
| **Any adverse event** - **no. of patients with adverse events (%)** | | | | | |
| Cardiovascular events t no. of patients (%) | 8/55 (15) | 8/56(14) | 13/71 (18) | 9/46 (20) | 0.30 |
| Gastrointestinal events^{‡}, no. of patients (%) | 4/55 (7) | 8/56 (14) | 9/71 (13) | 7/46 (15) | 0.54 |
| Hearing loss at least 15dB at ≥ 2 frequencies, no. of patients (%) | 10/44 (23) | 9/52 (17) | 14/63 (22) | 11/41 (27) | 0.26 |

| | | | | | |
|---|---|---|---|---|---|
| *P-value for the likelihood ratio test for treatment effect (eflornithine and sulindac vs. placebo) on adenoma recurrence in the full model which includes age, gender, race/ethnicity, aspirin usage, treatment, genotype, and treatment and genotype interaction as covariates. A statistically significant interaction was detected in the full model for adenoma recurrence (P=0.038); no interaction was detected for cardiovascular toxicity, gastrointestinal toxicity, or ototoxicity. ^{†}Cardiovascular events included coronary artery disease, myocardial infarction, cerebrovascular accident, congestive heart failure, and chest pain. ^{‡}Gastrointestinal events included gastrointestinal bleeding (from any region) such as rectal bleeding, upper gastrointestinal bleeding, hematochezia, or occult blood in the stool. | | | | | |

**Table 4. Efficacy and Adverse Events and the ODC1+316 SNP.**

| | Placebo (N = 111) | | | DFMO/Sulindac (N = 117) | | | *P* |
|---|---|---|---|---|---|---|---|
| | *ODC* GG (N=55) | *ODC* GA (N=48) | *ODC* AA (N=8) | *ODC* GG (N=71) | *ODC* GA (N=39) | *ODC* AA (N=7) | |
| Any adenoma recurrence (%) | 22/44 (50) | (35) | (29) | 7/64 (11) | (14) | (57) | < 0.0001 |
| Cardiovascular events no. of patients (%)a | 8/55 (15) | 7/48 (15) | 1/8 (13) | 13/71 (18) | 7/39 (18) | 2/7 (29) | 0.37 |
| Gastrointestinal events no. of patients (%)b | 4/55 (7) | 8/48 (17) | 0/8 (0) | 9/71 (13) | 5/39 (13) | 2/7 (29) | 0.45 |
| Hearing loss at least 15dB at ≥ 2 frequencies, no. of patients (%)c | 10/44 (23) | 9/45 (20) | 0/8 (0) | 14/63 (22) | 7/34 (21) | 4/7 (57) | 0.020 |

**Table 5. Characteristics of the Study Population at Baseline and at t Study* .**

| | **Baseline** n = 222 | **End-of-Study** n = 188 |
|---|---|---|
| **Mean Age** | | |
| (Years, w/ range) | 60 (47-75) | 60 (47-75) |

| **Gender** | | |
|---|---|---|
| Male | 164 (74%) | 141 (75%) |
| Female | 58 (26%) | 47 (25%) |

| **Ethnicity** | | |
|---|---|---|
| Caucasian | 192 (86%) | 160 (85%) |
| Hispanic | 12 (5%) | 12 (6%) |
| Black | 8 (4%) | 6 (3%) |
| Asian | 7 (3%) | 7 (4%) |
| Other | 3 (2%) | 3 (2%) |

| **Aspirin use** | | |
|---|---|---|
| Yes | 93 (42%) | 73 (39%) |
| No | 129 (58%) | 115 (61%) |

| **Number of Polyps** | | |
|---|---|---|
| 1 | 93 (42%) | 81 (43%) |
| 2 to 3 | 80 (36%) | 68 (36%) |
| >3 | 46 (21%) | 39 (21%) |

| **Polyp Size** | | |
|---|---|---|
| <5mm | 69 (31%) | 55 (29%) |
| 5-9 mm | 85 (38%) | 75 (40%) |
| 10-15 mm | 47 (21%) | 40 (21%) |
| >15 mm | 21 (10%) | 18 (10%) |

| **Polyp Location** | | |
|---|---|---|
| Rectum | 43 (19%) | 37 (20%) |
| Left Colon | 87 (39%) | 75 (40%) |
| Transverse colon | 31 (14%) | 25 (13%) |
| Right Colon | 35 (16%) | 30 (16%) |
| Cecum | 26 (12%) | 21 (11%) |

| **Polyp Histology** | | |
|---|---|---|
| Tubular | 170 (77%) | 143 (76%) |
| Tubulovillous | 30 (13%) | 25 (13%) |
| Villous | 6 (3%) | 6 (3%) |
| Cancer in situ | 2 (<1%) | 2 (1%) |
| High grade dysplasia | 1 (<1%) | 1 (<1%) |
| Adenoma Not classified | 13 (6%) | 11 (6%) |

| | | |
|---|---|---|
| *Values are count and column percentage for categorical variables, mean (range) for continuous variables | | |

**Table 6. Baseline Adenoma Characteristics by Dietary Polyamine Group (n=222).**

| | **Lower Dietary Polyamine Group n=167** | **Highest Dietary Polyamine Group n=55** | ***P**** |
|---|---|---|---|
| **Mean Number** | 2.61 (± 2.32 SD) | 2.41 (± 1.90 SD) | 0.98 |

| **Location** | | | |
|---|---|---|---|
| Right Sided (n=92) | 38.9 % | 51% | 0.20 |
| Left Sided (n=130) | 61.1% | 49% | |
| **Large Adenomas** | 26.4 % (n=44) | 43.6 % (n=24) | 0.016 |
| (≥ 1 cm) | | | |
| **High Grade Adenomas** | 20.4 % (n=34) | 32.7 % (n=18) | 0.060 |
| **Advanced Adenomas** | 35.9% (n=60) | 52.7% (n=29) | 0.028 |

| | | | |
|---|---|---|---|
| *Wilcoxon Rank Sum test *P* values are reported for the comparison of mean adenoma number; otherwise *P* values are reported using Chi-square tests for independence. | | | |

**Table 7. Tissue Polyamine Levels at Baseline by Dietary Polyamine Group.**

| **Mean Tissue Polyamines** (nmol/mg)* | **Lower Dietary Polyamine Group** n=167 | **Highest Dietary Polyamine Group** n=55 | ***P***** |
|---|---|---|---|
| Putrescine | 0.65 | 0.63 | 0.60 |
| Spermidine | 2.49 | 2.95 | 0.024 |
| Spermine | 7.90 | 8.92 | 0.039 |
| Spermidine:Spermine Ratio | 0.32 | 0.33 | 0.19 |

| | | | |
|---|---|---|---|
| *nmol polyamine per milligram protein **Wilcoxon Rank Sum test *P* values are reported | | | |

**Table 8. Colorectal Adenoma Recurrence Risk* after Treatment with DFMO+sulindac vs. Placebo, by Baseline Dietary Polyamine Group.**

| | | **Lower Dietary Polyamine Group (n=144)** | | | **Highest Dietary Polyamine Group (n=44)** | |
|---|---|---|---|---|---|---|
| | **n** | **Risk Ratio (95% Confidence Interval)** | ***P*** | **n** | **Risk Ratio (95% Confidence Interval)** | ***P*** |
| **Any Adenoma** | 45 | 0.19 (0.09-0.40) | 0.0001 | 9 | 1.04 (0.32-3.36) | 0.94 |
| **Adenomas >1cm** | 10 | 0.11 (0.01-0.88) | 0.03 | 2 | *** | *** |
| **High grade Adenomas** | 12 | 0.09 (0.01-0.71) | 0.02 | 1 | *** | *** |
| **Advanced Adenomas**** | 18 | 0.06 (0.00-0.44) | 0.005 | 3 | *** | *** |

| | | | | | | |
|---|---|---|---|---|---|---|
| * Relative risk estimation by log-binomial regression. Likelihood ratio test P values are reported. Risk ratios indicate risk of metachronous adenoma after treatment with DFMO+sulindac vs. placebo (referent group). All risk ratios are adjusted for aspirin intake ** Advanced adenoma is a variable that includes large adenoma size (> 1cm) and/or villous histology *** Insufficient power to calculate risk estimates | | | | | | |

### REFERENCES

The following references to the extent that they provide exemplary procedural or other details supplementary to those set forth herein.
U.S. Patent. 3,647,858
U.S. Patent. 3,654,349
U.S. Patent. 4,330,559
U.S. Patent. 4,413,141
U.S. Patent. 4,582,788
U.S. Patent. 4,656,127
U.S. Patent. 4,683,194
U.S. Patent. 4,683,202
U.S. Patent. 5,814,625
U.S. Patent. 5,843,929
U.S. Patent. 5,952,174
U.S. Patent. 6,258,845
Alberts et al., J. Cell. Biochem. Supp., (22):18-23, 1995..
AMA Drug Evaluations Annual, 1814-1815, 1994.
Babbar et al., Biochem. J., 394:317-24, 2006.
Babbar et al., J. Biol. Chem., 278(48):47762-47775, 2003.
Barry et al., J. Natl. Cancer Inst., 98(20):1494-500, 2006.
Bedi et al., Cancer Res., 55(9):1811-1816, 1995.
Bellofernandez et al., Proc. Natl. Acad. Sci. USA, 90:7804-8, 1993.
Bussey, Hepatology, 12(1):175-6. 1990
Childs et al., Cell. Molec. Life Sci., 60:1394-1406, 2003.
de Arruda et al., Expert Rev. Mol. Diagn., 2(5):487-496, 2002.
Derynck et al., Nature Genetics, 29:117-29, 2001.
DuBois et al., Cancer Res., 56:733-737, 1996.
Erdman et al., Carcinogenesis, 20:1709-13, 1999.
European Appln. 201,184
European Appln. 237,362
European Appln. 258,017
European Appln. 50,424
European Appln. 84,796
French Appln. 2,650,840
Fultz and Gerner, Mol. Carcinog., 34:10-8, 2002.
Gerner and Meyskens, Nature Rev. Cancer, 4:781-92., 2004.
Gerner et al., Cancer Epidemoil. Biomarkers Prev., 3:325-330, 1994.
Giardiello et al., Cancer Res., (57):199-201, 1997.
Guo et al., Cancer Res., 60(22):6314-6317, 2000.
Halushka et al., Nat. Genet., 22(3):239-247,1999.
Hanif et al., Biochemical Pharmacology, (52):237-245, 1996.
Hubner et al., Clin. Cancer Res., 14(8):2303-9, 2008.
Ignatenko et al., Cancer Biol. Ther., 5(12):1658-64, 2006.
Inazuka et al., Genome Res, 7(11):1094-1103, 1997.
Iwamoto et al., Carcinogenesis, 21:1935-40, 2000.
Johnson et al., Nat. Genet., 29(2):233-237, 2001.
Ke and Cardon Bioinformatics, 19(2):287-288, 2003.
Keller and Giardiello, Cancer Biol. Ther., 2(4 Suppl 1):S140-9, 2003.
Kingsnorth et al., Cancer Res., 43(9):4035-8, 1983.
Komher, et al., Nucl. Acids. Res. 17:7779-7784, 1989.
Kuppuswamy, et al., Proc. Natl. Acad. Sci. USA, 88:1143-1147,1991.
Kwok and Chen, Curr Issues Mol. Biol., Apr;5(2):43-60, 2003.
Kwok et al., Genomics, 23(1):138-144, 1994.
Kwok, Annu. Rev. Genomics Hum. Genet., 2:235-258, 2001.
Kwok et al., Genomics, 31(1):123-6, 1996.
Ladenheim et al., Gastroenterology, 108:1083-1087, 1995.
Landegren, et al., Science, 241:1077-1080, 1988.
Lanza et al., Arch. Intern. Med., 155:1371-1377, 1995.
Le et al., Cancer Epidemiol. Biomarkers Prev., 17:1950-62, 2008.
Lipkin, J. Cell Biochem. Suppl., 28-29:144-7, 1997.
Lippman, Nat. Clin. Pract. Oncol., 3(10):523, 2006.
Love et al., J. Natl. Cancer Inst., 85:732-7, 1993.
Lu et al., Eukaryot Cell., 3(6):1544-56, 2004.
Luk and Baylin, N. Engl. J. Med., 311(2):80-83, 1984.
Lupulescu, Cancer Detect. Prev., 20(6):634-637, 1996.
Martinez et al., Proc. Natl. Acad. Sci. USA, 100:7859-64, 2003.
Matsubara et al., Clinical Cancer Res., 1:665-71, 1995.
Maxam, et al., Proc. Natl. Acad. Sci. USA, 74:560, 1977.
McLaren et al., Cancer Prev. Res., 1(7):514-21, 2008.
Meyskens et al., Cancer Prev. Res., 1(1):32-8, 2008.
Meyskens et al., J. Natl. Cancer Inst., 86(15):1122-1130, 1994.
Meyskens et al., J. Natl. Cancer Inst., 90(16):1212-8, 1998.
Mullis et al., Cold Spring Harbor Symp. Quant. Biol. 51:263-273, 1986.
Muscat et al., Cancer, 74:1847-1854, 1994.
Narisawa et al., Cancer Res., 41(5):1954-1957, 1981.
Nickerson et al., Proc. Natl. Acad. Sci. USA, 87:8923-8927,1990.
Nyren et al., Anal. Biochem. 208:171-175, 1993.
O'Brien et al., Molec. Carcinog., 41(2):120-3, 2004.
Pardali and Moustakas, Biochimica et Biophysica Acta, 1775:21-62, 2007.
PCT Appln. WO91/02087
PCT Appln. WO92/15712
Peel et al., J. Natl. Cancer Inst., 92:1517-22, 2000.
Pegg, Biochem., 234(2):249-262, 1986.
Physician's Desk Reference, Medical Economics Data, Montville, N.J., 1745-1747, 1999
Piazza et al., Cancer Res., (55):311 3116, 1995.
Piazza et al., Cancer Res., (57):2452-2459, 1997a.
Piazza et al., Cancer Res., (57):2909-2915, 1997b.
Pollard and Luckert, Cancer Res., 49:6471-6473, 1989.
Prezant et al., Hum. Mutat., 1:159-164, 1992.
Psaty and Potter, N. Engl. J. Med., 355(9):950-2, 2006.
Rao et al., Cancer Res., (55):1464-1472, 1995.
Reddy et al., Cancer Res., (50):2562-2568, 1990.
Reddy et al., Cancer Res., 47:5340-5346, 1987.
Rice et al., Mol. Cancer Ther., 2(9):885-92, 2003.
Roberts and Wakefield, Proc. Natl. Acad. Sci. USA, 100:8621-3, 2003.
Sanger et al., J. Molec. Biol., 94:441, 1975.
Seiler and Knodgen, J. Chromatogr., 221(2):227-235, 1980.
Simoneau et al., Cancer Epidemiol. Biomarkers Prev., 17:292-9, 2008.
Simoneau et al., J. Natl. Cancer Inst., 93:57-9, 2001.
Singh and Reddy, Annals. NY Acad. Sci., (768):205-209, 1995.
Singh et al., Carcinogenesis, (15):1317-1323, 1994.
Small et al., N. Engl. J. Med., 347:1135-1142, 2002.
Sokolov, Nucl. Acids Res. 18:3671, 1990.
Stevens et al., Biotechniques, 34:198-203, 2003.
Strejan et al., Cell Immunol., 84(1):171-184, 1984.
Su et al., Science, (256):668-670, 1992.
Syvanen et al., Genomics 8:684-692, 1990.
Taillon-Miller et al., Genome Res, 8(7):748-754, 1998.
Tempero et al., Cancer Res., 49(21):5793-7, 1989.
Thomas and Thomas, J. Cell Mol. Med., 7:113-26, 2003.
Thompson et al., J. Natl. Cancer Inst., (87): 125-1260, 1995.
Ugozzoll et al., GATA 9:107-112, 1992.
Vane and Botting, Adv Exp Med Biol., 433:131-8, 1997.
Visvanathan et al., J. Urol., 171(2 Pt 1):652-5, 2004.
Wallace, Eur. J. Clin. Invest., 30:1-3, 2000.
Zell et al., Cancer Epidemiol. Biomarkers Prev., 17:3134-40, 2008.
Zell et al., Cancer Prev. Res., 2(3):209-12, 2009.
Zell et al., Clin. Cancer Res., 15(19):6208-16, 2009.
Zell et al., Intl. J. Cancer, 120:459-68, 2007.
Ziogas and Anton-Culver, Am. J. Prev. Med., 24:190-8, 2003.

## Claims

1. A composition comprising α-difluoromethylornithine (DFMO) and a spermidine/spermine *N*¹-acetyltransferase expression agonist that is a non-aspirin containing non-steroidal anti-inflammatory drug (NSAID), for use in the prevention or treatment of carcinoma in a patient,
wherein the dietary polyamine intake by said patient is less than 300 µmol polyamine per day.

2. A composition comprising α-difluoromethylornithine (DFMO) for use in the prevention or treatment of carcinoma in a patient,
wherein the dietary polyamine intake by said patient is less than 300 µmol polyamine per day; and
wherein the patient is also administered a composition comprising a spermidine/spermine *N*¹-acetyltransferase expression agonist that is a non-aspirin containing non-steroidal anti-inflammatory drug (NSAID), the patient being administered the composition comprising the non-aspirin containing NSAID before, at the same time as, or after the administration of the composition comprising DFMO; and
wherein, if the patient is administered the composition comprising the non-aspirin containing NSAID before, or after, the administration of the composition comprising DFMO, then the administration of the non-aspirin containing NSAID precedes or follows the administration of the composition comprising DFMO by an interval ranging from minutes to months.

3. A composition comprising a spermidine/spermine *N*¹-acetyltransferase expression agonist that is a non-aspirin containing non-steroidal anti-inflammatory drug (NSAID) for use in the prevention or treatment of carcinoma in a patient,
wherein the dietary polyamine intake by said patient is less than 300 µmol polyamine per day; and
wherein the patient is also administered a composition comprising α-difluoromethylornithine (DFMO), the patient being administered the composition comprising DFMO before, at the same time as, or after the administration of the composition comprising the non-aspirin containing NSAID; and
wherein if the patient is administered the composition comprising DFMO before, or after, the administration of the composition comprising the non-aspirin containing NSAID, then the administration of the DFMO precedes or follows the administration of the composition comprising the non-aspirin containing NSAID by an interval ranging from minutes to months.

4. The composition for use of any of claims 1-3, wherein the patient's dietary polyamine intake is determined from a patient dietary history.

5. The composition for use of any of claims 1-3, wherein the patient is subjected to a diet to reduce polyamine intake prior to or at the time of commencing said treatment.

6. The composition for use of any of claims 1-5, wherein said non-aspirin containing NSAID is selected from the group consisting of a selective COX-2 inhibitor, sulindac and celecoxib.

7. The composition for use of any of claims 1-6, wherein -
(i) the DFMO is administered orally, optionally wherein the effective amount of DFMO is 500 mg/day;
(ii) the DFMO is administered intravenously, optionally wherein the effective amount of DFMO is from 0.05 to 5.0 g/m²/day; or
(iii) the DFMO is administered at least a second time.

8. The composition for use of any of claims 1-6, wherein said non-aspirin containing NSAID is sulindac, and wherein the effective amount of sulindac is from 10 to 1500 mg/day, such as from 10 to 400 mg/day, or such as 150 mg/day; or wherein sulindac is administered at least a second time.

9. The composition for use of any of claims 1-6, wherein said patient has a solid tumor, and wherein said treatment further comprises resection of said solid tumor, and wherein DFMO and sulindac are administered prior to said resection, or wherein DFMO and sulindac are administered after said resection.

10. The composition for use of any of claims 1-9, wherein said carcinoma is colorectal cancer, neuroblastoma, breast cancer, pancreatic cancer, brain cancer, lung cancer, stomach cancer, a blood cancer, skin cancer, testicular cancer, prostate cancer, ovarian cancer, liver cancer or esophageal cancer, cervical cancer, head and neck cancer, non-melanoma skin cancer and glioblastoma.

11. The composition for use of any of claims 1-10, wherein the recurrence of carcinoma in a patient having been previously diagnosed with carcinoma is to be prevented, optionally wherein said patient has previously had a surgical resection of a carcinoma tumor.

12. The composition for use of any of claims 1-11, wherein said patient has been identified as having familial adenomatous polyposis.

13. A method of selecting a patient for suitability for treatment of carcinoma by the composition of any of claims 1-3 comprising assessing the dietary polyamine intake by said patient, wherein the patient is selected if the dietary polyamine intake by said patient is less than 300 µmol polyamine per day.

## Patentansprüche

1. Zusammensetzung, umfassend α-Difluormethylornithin (DFMO) und einen Spermidin/Spermin-*N*¹-Acetyltransferase-Expressionsagonisten, der ein nichtaspirinhaltiges nichtsteroidales entzündungshemmendes Arzneimittel (NSAID) ist, zur Verwendung bei der Vorbeugung oder Behandlung eines Karzinoms bei einem Patienten,
wobei die diätetische Polyaminaufnahme durch den Patienten weniger als 300 µmol Polyamin pro Tag beträgt.

2. Zusammensetzung, umfassend α-Difluormethylornithin (DFMO) zur Verwendung bei der Vorbeugung oder Behandlung eines Karzinoms in einem Patienten,
wobei die diätetische Polyaminaufnahme durch den Patienten weniger als 300 µmol Polyamin pro Tag beträgt; und
wobei dem Patienten auch eine Zusammensetzung verabreicht wird, die einen Spermidin/Spermin-*N*¹-Acetyltransferase-Expressionsagonisten umfasst, der ein nichtaspirinhaltiges nichtsteroidales entzündungshemmendes Arzneimittel (NSAID) ist, wobei dem Patienten die Zusammensetzung, die das nichtaspirinhaltige NSAID umfasst, vor, gleichzeitig oder nach Verabreichung der DFMO-umfassenden Zusammensetzung verabreicht wird; und
wobei, wenn dem Patienten die Zusammensetzung, die das nichtaspirinhaltige NSAID umfasst, vor oder nach Verabreichung der DMFO-umfassenden Zusammensetzung verabreicht wird, die Verabreichung des nichtaspirinhaltigen NSAID der Verabreichung der DMFO-umfassenden Zusammensetzung um ein Intervall im Bereich von Minuten bis Monaten vorausgeht oder auf diese folgt.

3. Zusammensetzung, umfassend einen Spermidin/Spermin-*N*¹-Acetyltransferase-Expressionsagonisten, der ein nichtaspirinhaltiges nichtsteroidales entzündungshemmendes Arzneimittel (NSAID) ist, zur Verwendung bei der Vorbeugung oder Behandlung eines Karzinoms bei einem Patienten,
wobei die diätetische Polyaminaufnahme durch den Patienten weniger als 300 µmol Polyamin pro Tag beträgt; und
wobei dem Patienten auch eine Zusammensetzung verabreicht wird, die α-Difluormethylornithin (DFMO) umfasst, wobei dem Patienten die DMFO-umfassende Zusammensetzung vor, gleichzeitig oder nach Verabreichung der Zusammensetzung, die das nichtaspirinhaltige NSAID umfasst, verabreicht wird; und
wobei, wenn dem Patienten die DMFO-umfassende Zusammensetzung vor oder nach Verabreichung der Zusammensetzung, die das nichtaspirinhaltige NSAID umfasst, verabreicht wird, die Verabreichung des DFMO der Verabreichung der Zusammensetzung, die das nichtaspirinhaltige NSAID umfasst, um ein Intervall im Bereich von Minuten bis Monate vorausgeht oder auf diese folgt.

4. Zusammensetzung zur Verwendung nach einem der Ansprüche 1 bis 3, wobei die dietätische Polyaminaufnahme des Patienten anhand einer Ernährungsanamnese des Patienten bestimmt wird.

5. Zusammensetzung zur Verwendung nach einem der Ansprüche 1 bis 3, wobei der Patient vor oder zu Beginn der Behandlung auf eine Diat gesetzt wird, um die Polyaminaufnahme zu verringern.

6. Zusammensetzung zur Verwendung nach einem der Ansprüche 1 bis 5, wobei das nichtaspirinhaltige NSAID aus der Gruppe ausgewählt ist, die aus einem selektiven COX-2-Inhibitor, Sulindac und Celecoxib besteht.

7. Zusammensetzung zur Verwendung nach einem der Ansprüche 1 bis 6, wobei -
(i) das DFMO oral verabreicht wird, wobei optional die wirksame Menge an DFMO 500 mg/Tag beträgt;
(ii) das DFMO intravenös verabreicht wird, wobei optional die wirksame Menge an DFMO 0,05 bis 5,0 g/m²/Tag beträgt; oder
(iii) das DFMO mindestens ein zweites Mal verabreicht wird.

8. Zusammensetzung zur Verwendung nach einem der Ansprüche 1 bis 6, wobei das nichtaspirinhaltige NASID Sulindac ist, und wobei die wirksame Menge an Sulindac 10 bis 1500 mg/Tag beträgt, wie beispielsweise 10 bis 400 mg/Tag oder wie beispielsweise 150 mg/Tag; oder wobei Sulindac mindestens ein zweites Mal verabreicht wird.

9. Zusammensetzung zur Verwendung nach einem der Ansprüche 1 bis 6, wobei der Patient einen soliden Tumor hat, und wobei die Behandlung ferner die Resektion des soliden Tumors umfasst, und wobei DFMO und Sulindac vor der Resektion verabreicht werden, und wobei DFMO und Sulindac nach der Resektion verabreicht werden.

10. Zusammensetzung zur Verwendung nach einem der Ansprüche 1 bis 9, wobei das Karzinom Kolorektalkrebs, Neuroblastom, Brustkrebs, Bauchspeicheldrüsenkrebs, Hirnkrebs, Lungenkrebs, Magenkrebs, ein Blutkrebs, Hautkrebs, Hodenkrebs, Prostatakrebs, Eierstockkrebs, Leberkrebs oder Speiseröhrenkrebs, Gebärmutterhalskrebs, Kopf-Hals-Krebs, Nicht-Melanom-Hautkrebs und Glioblastom ist.

11. Zusammensetzung zur Verwendung nach einem der Ansprüche 1 bis 10, wobei das Wiederauftreten eines Karzinoms bei einem Patienten, der zuvor mit Karzinom diagnostiziert wurde, zu verhindern ist, wobei optional der Patient zuvor einer chirurgischen Resektion eines Karzinomtumors unterzogen wurde.

12. Zusammensetzung zur Verwendung nach einem der Ansprüche 1 bis 11, wobei der Patient als familiäre adenomatöse Polyposis aufweisend identifiziert wurde.

13. Verfahren zum Auswählen eines Patienten hinsichtlich einer Eignung für eine Karzinombehandlung durch die Zusammensetzung nach einem der Ansprüche 1 bis 3, umfassend das Bewerten der dietätischen Polyaminaufnahme des Patienten, wobei der Patient ausgewählt wird, wenn die dietätische Polyaminaufnahme des Patienten weniger als 300 µmol Polyamin pro Tag beträgt.

## Revendications

1. Composition comprenant de l'a-difluorométhylornithine (DFMO) et un agoniste de l'expression de la spermidine/spermine *N*¹-acétyltransférase qui est un anti-inflammatoire non stéroïdien (AINS) ne contenant pas d'aspirine, destinée à être utilisée dans la prévention ou le traitement du carcinome chez un patient,
la prise alimentaire de polyamine par ledit patient étant inférieure à 300 µmol de polyamine par jour.

2. Composition comprenant de l'a-difluorométhylornithine (DFMO) destinée à être utilisée dans la prévention ou le traitement du carcinome chez un patient,
la prise alimentaire de polyamine par ledit patient étant inférieure à 300 µmol de polyamine par jour ; et
où l'on administre également au patient une composition comprenant un agoniste de l'expression de la spermidine/spermine *N*¹-acétyltransférase qui est un anti-inflammatoire non stéroïdien (AINS) ne contenant pas d'aspirine, la composition comprenant l'AINS ne contenant pas d'aspirine étant administrée au patient avant, pendant ou après l'administration de la composition comprenant de la DFMO ; et
où si l'on administre au patient la composition comprenant l'AINS ne contenant pas d'aspirine avant ou après l'administration de la composition comprenant de la DFMO, alors l'administration de l'AINS ne contenant pas d'aspirine précède ou suit l'administration de la composition comprenant de la DFMO selon un intervalle allant de quelques minutes à quelques mois.

3. Composition comprenant un agoniste de l'expression de la spermidine/spermine *N*¹-acétyltransférase qui est un anti-inflammatoire non stéroïdien (AINS) ne contenant pas d'aspirine, destinée à être utilisée dans la prévention ou le traitement du carcinome chez un patient,
la prise alimentaire de polyamine par ledit patient étant inférieure à 300 µmol de polyamine par jour ; et
où l'on administre également au patient une composition comprenant de l'α-difluorométhylornithine (DFMO), le patient se voyant administrer la composition comprenant la DFMO avant, pendant ou après l'administration de la composition comprenant l'AINS ne contenant pas d'aspirine ; et
où si l'on administre au patient la composition comprenant de la DFMO avant ou après l'administration de la composition comprenant de l'AINS ne contenant pas d'aspirine, alors l'administration de la DFMO précède ou suit l'administration de la composition comprenant l'AINS ne contenant pas d'aspirine selon un intervalle allant de quelques minutes à quelques mois.

4. Composition destinée à être utilisée selon l'une quelconque des revendications 1 à 3, la prise alimentaire de polyamine par le patient étant déterminée par l'anamnèse alimentaire du patient.

5. Composition destinée à être utilisée selon l'une quelconque des revendications 1 à 3, le patient étant soumis à un régime alimentaire destiné à réduire la prise de polyamine avant ou au moment de commencer ledit traitement.

6. Composition destinée à être utilisée selon l'une quelconque des revendications 1 à 5, ledit AINS ne contenant pas d'aspirine étant sélectionné dans le groupe constitué d'un inhibiteur de COX-2 sélectif, de sulindac et de célécoxib.

7. Composition destinée à être utilisée selon l'une quelconque des revendications 1 à 6, -
(i) la DFMO étant administrée par voie orale, la quantité efficace de DFMO étant éventuellement de 500 mg/jour ;
(ii) la DFMO étant administrée par voie intraveineuse, la quantité efficace de DFMO étant éventuellement de 0,05 à 5,0 g/m²/jour ; ou
(iii) la DFMO étant administrée au moins une deuxième fois.

8. Composition destinée à être utilisée selon l'une quelconque des revendications 1 à 6, ledit AINS ne contenant pas d'aspirine étant le sulindac et la quantité efficace de sulindac étant de 10 à 1 500 mg/jour, par exemple de 10 à 400 mg/jour ou par exemple de 150 mg/jour ; ou le sulindac étant administré au moins une deuxième fois.

9. Composition destinée à être utilisée selon l'une quelconque des revendications 1 à 6, ledit patient présentant une tumeur solide, ledit traitement comprenant en outre une résection de ladite tumeur solide, et la DFMO et le sulindac étant administrés avant ladite résection ou la DFMO et le sulindac étant administrés après ladite résection.

10. Composition destinée à être utilisée selon l'une quelconque des revendications 1 à 9, ledit carcinome étant un cancer colorectal, un neuroblastome, un cancer du sein, un cancer du pancréas, un cancer du cerveau, un cancer du poumon, un cancer de l'estomac, une leucémie, un cancer de la peau, un cancer des testicules, un cancer de la prostate, un cancer de l'ovaire, un cancer du foie ou un cancer de l'oesophage, un cancer du col de l'utérus, un cancer de la tête et du cou, un cancer de la peau non mélanome ou un glioblastome.

11. Composition destinée à être utilisée selon l'une quelconque des revendications 1 à 10, la récidive du carcinome devant être empêchée chez un patient chez qui un carcinome a été diagnostiqué par le passé, ledit patient ayant éventuellement déjà subi une résection chirurgicale d'une tumeur du carcinome.

12. Composition destinée à être utilisée selon l'une quelconque des revendications 1 à 11, ledit patient ayant été identifié comme présentant une polypose adénomateuse familiale.

13. Procédé de sélection d'un patient apte au traitement du carcinome au moyen de la composition selon l'une quelconque des revendications 1 à 3 comprenant l'évaluation de la prise alimentaire de polyamine par ledit patient, le patient étant sélectionné si la prise alimentaire de polyamine par ledit patient est inférieure à 300 µmol de polyamine par jour.
